(19) Europäisches Patentamt
European Patent Office
Office européen des brevets

(11) **EP 3 573 068 A1**

(12) **EUROPEAN PATENT APPLICATION**

(43) Date of publication:
**27.11.2019 Bulletin 2019/48**

(51) Int Cl.:
**G16H 10/60** (2018.01)

(21) Application number: **18187263.1**

(22) Date of filing: **03.08.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.05.2018 EP 18174108
29.06.2018 EP 18180907**

(71) Applicant: **Siemens Healthcare GmbH
91052 Erlangen (DE)**

(72) Inventor: **Vodencarevic, Asmir
90763 Fürth (DE)**

Remarks:
Amended claims in accordance with Rule 137(2)
EPC.

(54) **SYSTEM AND METHOD FOR AN AUTOMATED CLINICAL DECISION SUPPORT SYSTEM**

(57) The invention describes a method for creating predictive models for an automated clinical decision support system for automated supervised and semi-supervised classification and treatment optimization of clinical events, e.g. of disease activity in autoimmune diseases, using EMR data and predictive models in a nested cross validation, as well as a respective prediction-unit for creating prediction-data for an automated clinical decision support system. The invention also describes a method for automated clinical decision support for automated supervised and semi-supervised classification and treatment optimization of clinical events using EMR data, as well as a respective decision support system.

FIG 23

EP 3 573 068 A1

**Description**

The invention describes a method for creating predictive models for an automated clinical decision support system for automated supervised and semi-supervised classification and treatment optimization of clinical events, e.g. of disease activity in autoimmune diseases, using EMR data, as well as a prediction-unit for creating prediction-data for an automated clinical decision support system. The invention also describes a method for automated clinical decision support for automated supervised and semi-supervised classification and treatment optimization of clinical events using EMR data, as well as a respective clinical decision support system.

Background information

Electronic Medical Records (EMRs) of patients represent systematized collections of patients' health data. They are populated in a clinical routine in a longitudinal manner through interactions of the patients with healthcare providers. Electronic medical records can contain different types of data, comprising demographic data, laboratory measurements over time, administered medications, examination data, clinical notes and images (e.g. from X-ray imaging, from ultrasound devices, from computed tomography or magnetic resonance imaging). On the patient population level (e.g. patient population diagnosed with a specific autoimmune disease such as Rheumatoid Arthritis (an acronym is "RA")), electronic medical records are large data repositories that can be utilized by data-driven approaches such as machine learning classification algorithms in various practical use cases. One exemplary use case is using the EMR data of patients diagnosed with RA to estimate the probability of flares within a certain time horizon (e.g. 3 months or 6 months). Such data-driven approaches require the data to be in a tabular format with columns representing different variables (e.g. patient age, gender, lab measurements) and rows representing patient follow-ups during which various data is collected. Moreover, the data has to be complete (i.e. missing values have to be treated in some way) and the label has to be available (i.e. whether a flare has occurred or not within an observed time horizon).

In general there are different problems for applying data-driven approaches to electronic medical records:

(A) The EMR data is collected at irregular time intervals which are different for different patients (some patients have many more follow-ups than others). Not all in the EMR available fields/variables are collected/measured/entered at each follow-up resulting in a scarcely populated EMR (i.e. many missing values). In the RA example, this is especially the case with many relevant variables such as the CRP laboratory measurement. Such scenario makes it impossible to apply statistical or machine learning approaches to predictive modeling as they rely on data in a tabular format and most of them cannot work directly with the missing values. Moreover, even if a value is available for the current follow-up, it is temporally related to previously observed values that should be analyzed in the common context.

(B) EMRs often contain hidden structures in the data which have predictive power with respect to some classification task (e.g. predicting RA flare occurrence within a certain time horizon). However, these structures are not obvious or directly accessible in the original multidimensional data space and therefore cannot be used explicitly in the modeling task.

(C) Electronic medical records can include many variables making it difficult to keep an overview of the disease dynamics, i.e. changes in a specific disease activity over time.

(D) When using electronic medical records of a patient population for building a classifier to predict the future disease activity (e.g. building classifiers for predicting the flare probability in RA patients), the follow-ups need to include a label about the disease activity (in the RA example the label shows whether the flares are observed within a certain time horizon after that follow-up or not) which is often not available or expensive/time-consuming to obtain. In some observed real-world cases, the label is missing in about 2/3 of the follow-ups.

(E) When building classifiers for autoimmune diseases from the EMR data, no free lunch theorem holds, i.e. there is no way to know a priori which type of an algorithm and with what hyperparameter values would work the best on the given problem. This causes long development times of such classifiers as various options need to be evaluated and compared. By using certain model selection procedures it would be possible to partially automate this model selection procedure; however it is not obvious how such procedure could work when the available EMR datasets contain both labeled and unlabeled data.

(F) Clinicians treating autoimmune diseases don't have an insight in trade-offs between various predicted risks of adverse events (such as e.g. RA flares or even death within different time horizons) and expenses associated with their treatment decisions including follow-up frequency. With such trade-offs, an implementation of the clinical decision support system giving an optimal treatment recommendations would be possible. Moreover, different clinicians prefer different treatment strategies, i.e. some are more conservative and some more liberal in treating the disease which makes it hard to establish a common generally acceptable standard for treatment recommendations.

Various approaches are known for the person skilled in the art in order to deal with the described problems:

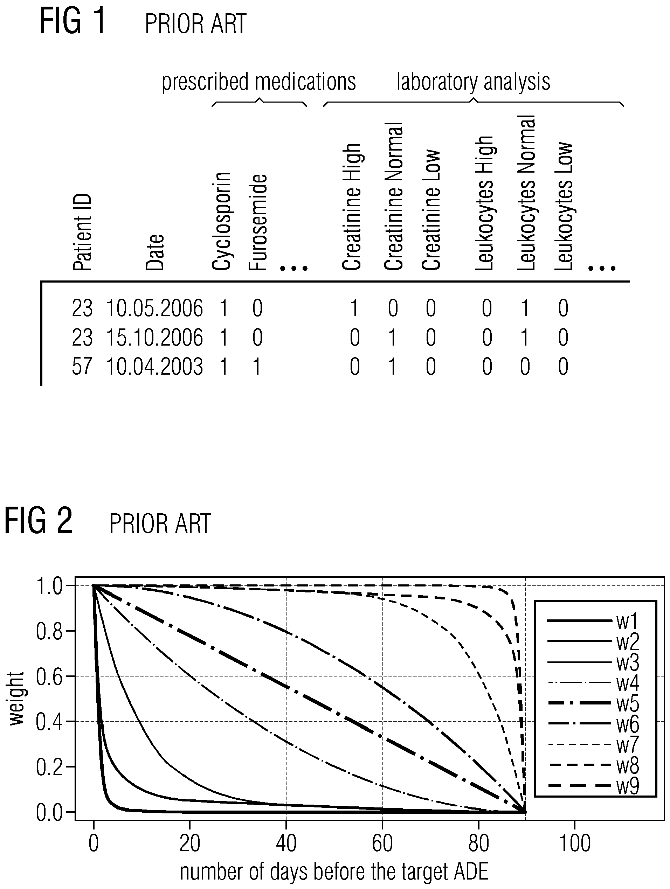

(A) It is known to treat missing values by imputation methods such as mean, median, regression imputation and multiple imputations, where missing values are estimated using known values. Another approach is to encode the missing values by binarization. If the variable is categorical with N levels (e.g. gender has N=2 levels: male and female), binarization approach creates two binary variables where the first one has the value 1 for those patients who are male and zero for those who are female. Likewise, the second variable has the value 1 for the female patients and value 0 otherwise. In case the value is missing for a patient, his/her gender is encoded with values 0 in both binary variables. If the variable is numerical, one or more cutting points are defined based on some statistics, e.g. based on the median value (or based on 33% and 67% percentiles for two thresholds or similar). For a threshold based on these percentiles, three binary variables are created, the first one containing values '1' at positions where corresponding numerical value is lower than or equal to the 33% percentile (measurement low value),the second one containing '1' where corresponding numerical value is higher than 33% percentile and lower or equal to the 66% percentile (measurement normal value), and the third one containing '1' where the corresponding numerical value is higher than the 66% percentile. If the numerical value is missing, all three binary variables receive value 0. Fig. 1 illustrates this approach used to encode categorical medication variables as well as the numerical laboratory values.
Temporal dependencies of clinical events or measurements can be modeled using weighting functions within sliding windows. Namely, a time frame is defined based on the relevancy of the past data for the current disease activity. This time frame represents the length of the sliding window that defines the weighting function. Such function has to be monotonically decreasing (e.g. linearly or exponentially, see Fig. 2), having its maximum value of 1 at the most recent follow-up recorded in the patient's EMR and its minimum value of 0 at the last time point within the relevancy time frame. For each current visit, the currently measured value is replaced by a sum of relevant past values multiplied by their corresponding weights. However, from the prior art it is unknown how to address the missing value problem, and in general the applied method produces unrealistic and for clinicians uninterpret-able feature values (e.g. typical CRP blood value could be replaced by a weighted sum of relevant past values which can be easily outside of the possible CRP values).

(B) It is known to apply clustering to identify subgroups / hidden structures of patients from their EMR data. However clustering EMR follow-ups of patients with certain disease in order to compute cluster features and them in predictive modeling of a disease activity has not been applied to the EMR data in the prior art.

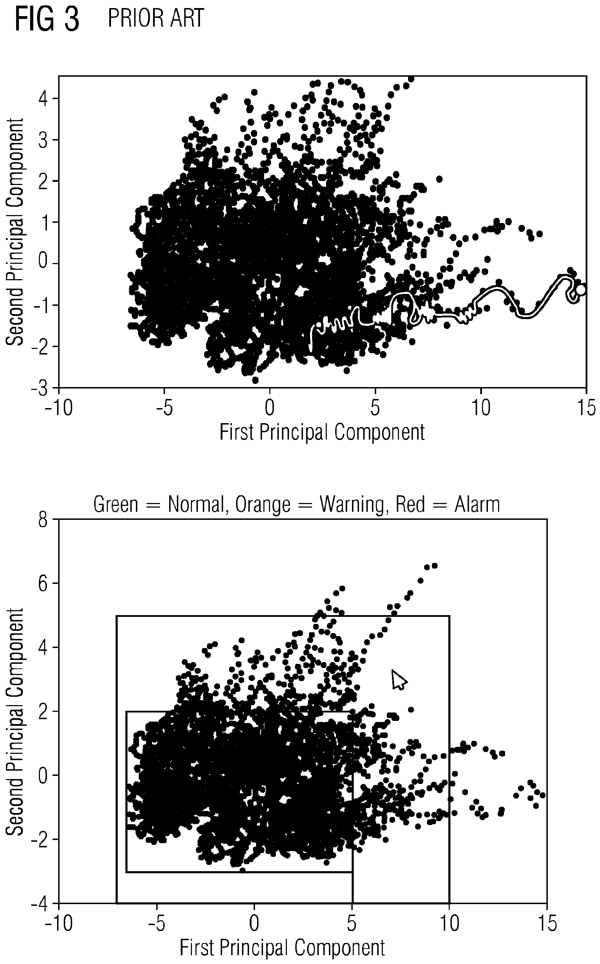

(C) It is known to apply dimensionality reduction using methods such as Principal Component Analysis (PCA) for the purpose of visualization to the EMR. PCA is a technique for dimensionality reduction that can reduce the EMR data to two or three dimensions (called principal components), which can be easily visualized. However, in the known prior art this is done in a static way, i.e. each visualized data point represents one patient in a new PCA space. This enables visualization of patients; however this visualization is static, not revealing changes in patients' health over time. It is known to use the PCA approach in a dynamic way in other technical areas, namely in predictive maintenance of technical systems such as gas turbine engines and jet engines, which is illustrated in Fig. 3.

(D) It is known to treat follow-ups with missing labels by excluding them from the analysis. However, this can cause big loss of the valuable data. Furthermore it is known to treat missing labels in patient EMRs by denoising autoencoders. In this setting, both available labeled and unlabeled data are used to train a denoising autoencoder (a type of deep learning neural network). The unlabeled data is then discarded and the hidden layer of the denoising autoencoder is used as the input to classical supervised learning methods such as a random forest. Therefore, in this approach the unlabeled instances are NOT labeled but rather used in an unsupervised manner to improve tuning neurons in the hidden layer of the denoising autoencoder. Moreover, since hidden layer performs dimensionality reduction it provides abstract features to the classifier, which are not understandable to or interpretable by clinicians.

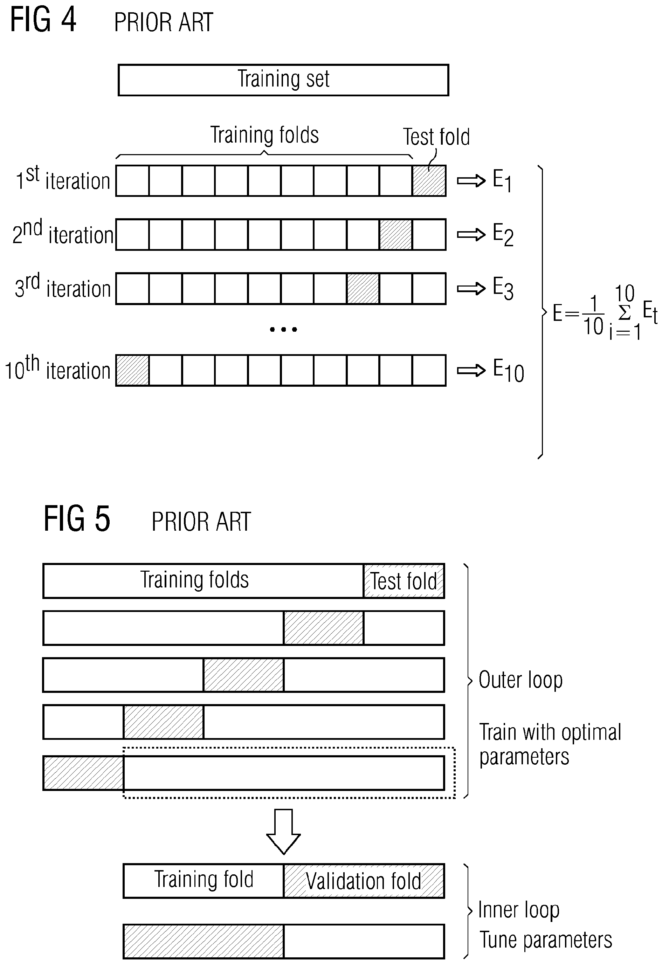

(E) It is known to perform selection and hyperparameter optimization in predictive modeling using a procedure called k-fold cross-validation. In this procedure, the available dataset is divided in k mutually exclusive partitions, mostly by random sampling without replacement. In the first fold, k-1 partitions are used for training a model which is then evaluated on the remaining partition. This is repeated k times, each time having different k-th set as a test set. In each fold, some performance metric is computed, such as the Area Under the receiver operating Characteristics (AUC) or classification error. Afterwards, the mean value and the standard deviation of the performance metrics are computed over k folds. For each model and for each set of hyperparameters such a k-fold cross validation procedure is performed, averaged results of the k-folds are compared for different models (and/or hyperparameter sets) and finally the one that maximized the model performance is selected as the best model. The illustration of the k-fold cross-validation is given in Fig. 4.

However, the performance computed in this way can be significantly biased, as the results on the k-test sets are actually used to make decisions about models and/or hyperparameter values (i.e. it is known that the problem of overfitting can occur). In order to mitigate this problem, a nested k-fold cross-validation procedure can be used, which provides an almost unbiased estimate of the true performance on unseen data (see e.g. Semi-Supervised Learning; edited by Olivier Chapelle, Bernhard Schölkopf, and Alexander Zien). In short, the nested cross-validation consists of two k-fold loops (note that loops can have different values of the 'k' parameter): the inner one which tunes the hyperparameters and the outer one which estimates the performance. The application of the nested cross-validation for model selection and hyperparameter optimization is straightforward: a number of supervised learning algorithms and their corresponding hyperparameter values are defined in a grid and evaluated in a nested cross-validation procedure w.r.t. some performance metrics of interest. At the end, the best model and its parameter set is selected. The procedure is illustrated in Fig. 5.

(F) It is known to compare a therapy selected by a clinician or a nurse to the therapy recommended by the proposed clinical decision support system based on the machine learning model (i.e. the output of the machine learning model is a therapy recommendation). Applied machine learning modeling formalism is the recurrent neural network. In this approach, the most likely therapy is directly modeled as an output of the machine learning task and moreover, the considered optimization problem relates to the therapy type only without considering additional factors such as the therapy expense and its influence on the patient follow-up frequency. Furthermore, multi-objective optimization is known which takes into account the following criteria: therapy timing, type and expense with constraints imposed on the type and maximum allowed expense. It is a numerical optimization problem which doesn't rank the importance of these single optimization criteria for clinicians. Moreover, it doesn't provide insights into trade-offs between these criteria.

Concerning EMR-data, there is the disadvantage that there does not exist a method or system that is able to process EMR-datasets automatically.

So it is the object of the present invention to improve the known methods for automatically making predictions based on electronic medical records.

This object is achieved by the method for creating predictive models for an automated clinical decision support system for automated supervised and semi-supervised classification and treatment optimization of clinical events using EMR data of claim 1; by the prediction-unit for creating prediction-data for an automated clinical decision support system of claim 8; by the method for automated clinical decision support of claim 9; by the Clinical Decision Support System of claim 12; and by the Data processing system of claim 13.

In the following, the invention may be described using examples with respect to predicting the probability of flares of Rheumatoid Arthritis, but the invention is not limited to this application. The invention and is aspects can be used in particular for predicting the future status of a patient having a certain disease, in particular an auto-immune disease.

The method of the invention for creating predictive models for an automated clinical decision support system for automated supervised and semi-supervised classification and treatment optimization of clinical events using EMR data is especially applicable (or even designed) for evaluating disease activity in autoimmune diseases or hospital readmissions. The method comprises the following steps:

-    Providing EMR-datasets.
     A (vast) number of EMR-datasets comprising measurements and patient related data of a number of follow-ups is provided. It should be noted although EMR datasets may comprise only measurements, usually not all data collected during follow-ups are measurements. Some data are e.g. patient selfassessment score or demographic data like gender and postal code.
-    Treating EMR-datasets (preferred).
     The EMR-datasets are treated in order to estimate missing values and/or to correct outliers and/or to model temporality of measurements. Preferably, new values are calculated (especially in or with moving windows) simulating missing values or replacing existing values. The modeling of temporality is advantageous to render different datasets with different time-scales in a way that they are comparable.
-    Forming subgroups (preferred).
     Subgroups of related follow ups are formed within the EMR-datasets, in order to extract special features from the data. This forming of subgroups (or "clusters") is explained further below.

- Providing a target-variable (preferred, however necessary for many applications).
  This target-variable can be provided manually or extracted directly from the EMR-dataset. The expression "target variable" (also called "output variable" or (mostly in statistics) "dependent variable" or "outcome variable") is well known in the technical field of KI for a variable that should be estimated.

The provision of the target variable is very advantageous and partly a necessity for a number of preferred embodiments explained below. In the case the predictive model is not implicitly aligned to a target, the target could be provided explicitly by labeling EMR-datasets. The target could be a label assigned to a group of EMR-datasets, e.g. "is there the possibility of a disadvantageous event occurring to the patient". The provision or extraction of the target variable should be performed whenever possible.

In the case, the target could be extracted from the EMR-datasets they could be referred to as "labeled EMR-datasets". In the case, the target could not directly be extracted from the EMR-datasets they could be referred to as "unlabeled EMR-datasets".

- Providing predictive models
  A number of untrained predictive models are provided (especially chosen from a predefined set of models, including e.g. RandomForest, AdaBoost, Logistic Regression etc.). The predictive models are able to output probabilities and assign weights to EMR-data, wherein each model is capable of being trained with data using methods of machine learning.

- Providing time horizons.
  A number of different time horizons (e.g. 1 month, 3 months, etc.) are provided. It is one goal of the invention, to create one final best model for each time horizon.

- Performing a nested cross-validation for each time horizon and for each predictive model.

- Selecting a predictive model for a time horizon based on the nested cross-validation.

A prediction-unit of the invention for creating prediction-data for an automated clinical decision support system for Method for automated clinical decision support for automated supervised and semi-supervised classification and treatment optimization of clinical events using EMR data comprises a number of trained prediction models for different time horizons (especially one for each different time horizon) as created by the above method.

A method of the invention for automated clinical decision support for automated supervised and semi-supervised classification and treatment optimization of clinical events using (new) EMR data is explained in the following. In practice, actual EMR-datasets from a follow-up are used, where there are normally no targets (labels) created yet. For example, in a case the label would indicate that a certain event occurs X month after the follow-up, this label cannot be available to the present time of the follow-up, since it will only be known in future. The Method comprises the following steps:

- Providing trained prediction models for a number of time horizons, especially trained by the above method. Especially a prediction unit of the invention can be used. There is especially one single prediction model for each time horizon.

- Providing an (especially unlabeled) EMR-dataset of a patient comprising measurements and patient related data of a number of follow-ups including the data of a present patient follow-up.

- (Preferred:) Treating the EMR-datasets in order to estimate missing values and/or to correct outliers and/or to model temporality of measurements. This treatment is very advantageous, however only if the EMR-datasets are not flawless. Nevertheless, even if there are flawless EMR-datasets, the method is preferably designed to apply this step, for the case there are EMR-datasets that are not flawless.

- (Preferred:) Form subgroups of related data given at different follow-ups within the EMR-datasets and extract cluster (subgroup) features. If used in the model training, clusters (subgroups) are especially provided that are derived in the training phase to extract cluster features.

- (Preferred:) Reduce the number of data dimensions in the EMR-datasets and visualizing the EMR-datasets in reduced number of dimensions. Especially visualizing data of the EMR-dataset in reduced number of dimensions derived in the training phase.

- Calculate the probability of a clinical event in all relevant time horizons with the number of trained prediction models. This is done especially for different treatment options, e.g. for different medication and/or doses.

In addition, the calculated probability of a clinical event in all relevant time horizons could be displayed or otherwise provided for a user or a using system.

A Clinical Decision Support System of the invention for automated supervised and semi-supervised classification and treatment optimization of clinical events using EMR data, comprises a prediction-unit of the invention. The Decision Support System is designed to execute a method of the invention for automated clinical decision support for automated supervised and semi-supervised classification and treatment optimization of clinical events using EMR data and to visualize the calculated results.

A data processing system of the invention, that is especially a computer network system, comprises a data-network, a number of client computers and a service computer-system, wherein the service computer system comprises a Clinical Decision Support System of the invention for automated supervised and semi-supervised classification and treatment optimization of clinical events using EMR Data.

The units or modules of the invention mentioned above can be completely or partially realised as software modules running on a processor of a computing system. A realisation largely in the form of software modules can have the advantage that applications already installed on an existing system can be updated, with relatively little effort, to install and run the methods of the present application. The object of the invention is also achieved by a computer program product with a computer program that is directly loadable into the memory of a computing system and which comprises program units to perform the steps of the inventive method when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

Particularly advantageous embodiments and features of the invention are given by the dependent claims, as revealed in the following description. Features of different claim categories may be combined as appropriate to give further embodiments not described herein.

The invention combines solutions to several aspects of the above mentioned problems (A) to (F). In the following, the invention is explained by explicitly describing how problems (A) to (F) are solved by the inventive concept. In addition, preferred solutions are described. Especially, some of the following embodiments could be for themselves or combined with features of other embodiments an individual invention.

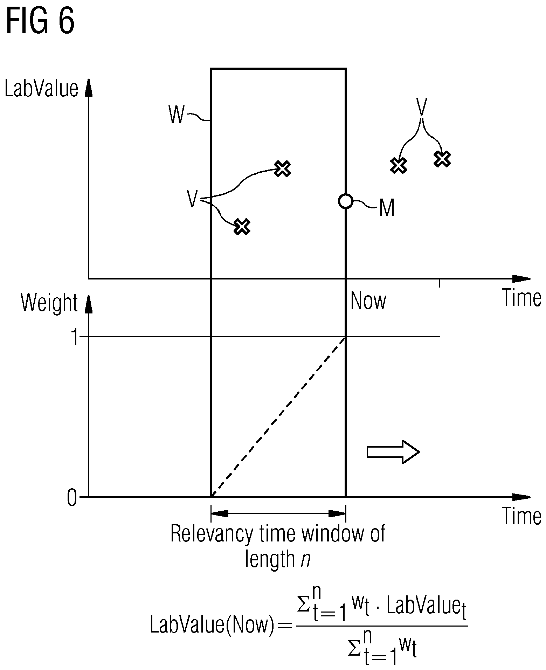

(A) According to an aspect of the invention, a sliding weighting function is used to estimate missing values in numerical variables. As long as there are some measurements in the relevancy time window, missing values are in particular estimated from them as the sum of weighted past known values. Since such sums can result in values which are not interpretable by clinicians, according to an aspect of the invention normalization by the sum of weights is used. In this way the estimated values will remain within typical and for clinicians understandable ranges of the corresponding measurements and moreover, the temporality of the measurements is modeled explicitly by value aggregation. In order to fully account for the temporality in the data, known measurements can be replaced by the aggregated ones. This normalized temporal aggregation step is illustrated in Fig. 6.

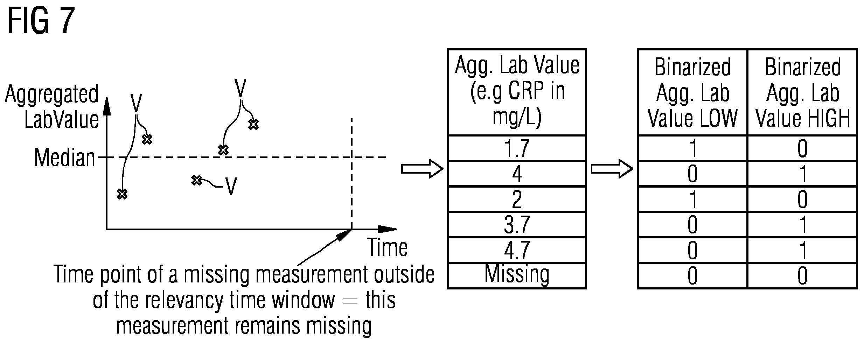

However, if there are no known values within the relevancy time frame, in general such estimation is not possible, i.e. if all previous values within the relevancy time window are missing, the current value will remain missing. Still, with this approach the number of missing values in the EMR is significantly reduced. After performing the normalized temporal aggregation, according to a further aspect of the invention a binarization approach is applied to encode the remaining missing values as binary zero vectors as described above. Since the number of missing values is already reduced, smaller portion of values will be encoded by dummy binary variables having zero value. This is illustrated in Fig. 7.

In a preferred method, missing values in the EMR-datasets are estimated by using a sliding weighting function, wherein especially missing values are estimated from measurements of the EMR-datasets in a relevancy time window as a sum of weighted past known values, preferably by normalizing this sum by the sum of weights.

Alternatively or additionally, the temporality of measurements in the EMR-datasets is modeled explicitly by value aggregation, wherein in order to fully account for the temporality in the data, known measurements are preferably replaced by the aggregated values.

In a preferred embodiment, after performing one of the afore described steps (especially both steps), in a "normalized temporal aggregation", a binarization approach is preferably applied to encode the potentially remaining missing values as binary zero vectors.

(B) As described above, detecting and visualizing subgroups within the EMR data using clustering approaches is known in the prior art. To that aim, various well-known clustering algorithms such as e.g. a k-means algorithm are used. According to a further aspect of the invention these hidden groups exploited to generate features (i.e. predictors), which might be predictable with respect to the given classification task. In particular, the follow-ups of patients are clustered using some clustering algorithm (e.g. k-means). The optimal number of clusters for the given dataset is determined using known methods, such as the silhouette analysis. Once the hidden groups (i.e. clusters) of patients' follow-ups are obtained, according to a further aspect of the invention they are used as predictors in the classification of disease activity (i.e. flare prediction, but this is generalizable to other disease data) in the following way: for each follow-up the cluster membership becomes one feature and for each follow-up the Minkowski distance (e.g. Euclidean) to the center of each cluster (called a centroid) becomes an additional feature. In this way, the following number of additional predictors is created: (number of clusters + 1).

In a preferred method, the subgroups of related data are formed from different follow-ups within the EMR data by using a clustering algorithm, such as e.g. a k-means algorithm. These subgroups are especially detected and visualized.

In a preferred embodiment, these subgroups are exploited to generate predictors, particularly by clustering follow-ups of patients in the EMR-datasets to subgroups, especially by using some clustering algorithm like k-means. The optimal number of subgroups for a given EMR-dataset is determined, preferably by using silhouette analysis.

The subgroups are used as predictors in the classification of clinical events, e.g. flare prediction in RA, in that for each follow-up the following steps are performed:

- A first feature is created from the cluster membership of the follow-up,
- Other features are created from the Minkowski distance of the follow-up (e.g. Euclidean) to the center of each subgroup (called a centroid).

For example, if there are three subgroups, a feature (may also be called "Variable" or "Predictor") defining the distance to the center of each subgroup is generated. In total tree features are generated in this example. In this way, the following number of additional predictors is created: (number of clusters + 1).

(C) Patients are data generators just like many technical systems. Therefore, in order to easily monitor changes in their health conditions based on the data collected at their follow-ups, according to a further aspect of the invention approaches from the area of predictive maintenance of technical systems are used. In particular, the PCA-based condition monitoring approach can be used. Multidimensional EMR patient data is represented in the PCA space using two or maximum three dimensions (so that it can still be visualized and understood by clinicians). All those follow-ups that are associated with the occurrence an event of interest (in the RA use case this is a flare) are represented in the PCA space as points of one color and all other follow-ups as points of another color. These points are likely to fall in two more or less overlapping groups. At each patient visit, when new data is collected, it will be mapped as a point into the PCA space where data of all previous visits of this patient are already marked in the order of appearance. This can be implemented in the animated manner.

In a preferred method, the number of data dimensions in the EMR-datasets is reduced and the EMR-datasets in reduced number of dimensions are visualized. For clarity it is noted that other steps of the inventive method may be performed in the original dimensions.

The number of data dimensions in the EMR-datasets is preferably reduced by using a Principal Component Analysis ("PCA"), especially a PCA-based condition monitoring approach. Patient data of the EMR-datasets (that are often multidimensional) are represented in a PCA-space using two or three dimensions. Data from follow-ups that are associated with a clinical event (in the RA use case this would be a flare) are represented in the PCA-space as points of one individual subgroup. Data from other clinical events form another subgroup (e.g. by color-coding). The subgroups are preferably visualized, especially in a space, where dangerous areas (i.e. areas where there are dangerous conditions for a patient) are marked.

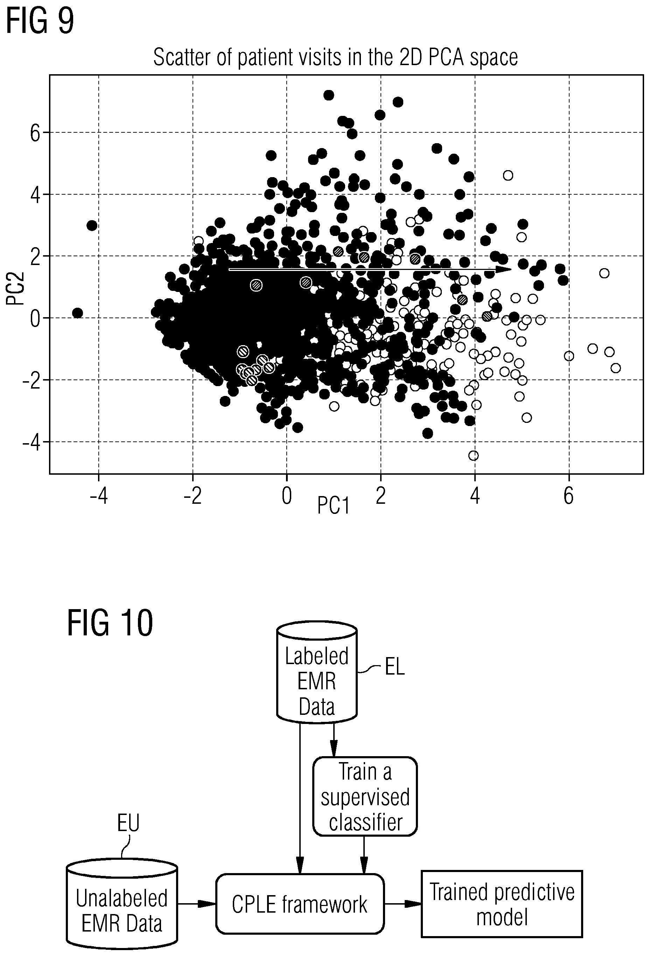

(D) In contrast to applying denoising autoencoders to make use of the unlabeled EMR patient data (the label can be for example disease activity or flare occurrence in rheumatoid arthritis), according to an aspect of the invention the Contrastive Pessimistic Likelihood Estimation framework (an acronym is "CPLE") is used. Denoising autoencoders use the unlabeled data only in the pre-training step, to tune the hidden network layer which reduces dimensionality of the EMR data. Then the unlabeled data is discarded and only the labeled data is provided to the trained hidden layer, which reduces its dimensionality and provides the abstract features to a supervised learning algorithm such as the random forest. Under the CPLE framework, the typical assumptions of the semi-supervised approaches such as the smoothness and the clustering assumption are not required. The CPLE includes the original supervised learning solution (i.e. a classifier trained on the labeled data only) explicitly into the objective function which is optimized, assigning "soft" labels to the unlabeled data. In this way, the potential improvements of the solution are controlled, i.e. the resulting classifier should not perform worse than the one trained on the labeled data only. The amount of improvement depends on the dataset itself as well as on the ratio of the labeled and unlabeled data. Typically, when the number of labeled instances is large, the inclusion of unlabeled instances rarely brings significant improvements and vice versa. With small adjustments, other semi-supervised algorithms (like transductive support vector machines or self-learning classifiers) can be used as well. CPLE can work with any supervised classifier that (a) allows instance weighting and (b) can output probability estimates. The CPLE framework is illustrated in Fig. 10. A preferred method concerns the case that the EMR-datasets comprise unlabeled data. In this case, the inner loop optimal model of the nested cross-validation is retrained using a Contrastive Pessimistic Likelihood Estimation framework assigning soft labels to unlabeled data. For labeling unlabeled data in the EMR-dataset the following steps are preferably applied:

- Create a supervised classifier.
  This supervised classifier is created by training and/or tuning based on labeled EMR-data from the EMR-dataset and a predefined grid of hyperparameter values, by using the inner loop of a nested cross validation routine.
- Choosing soft-labels.
  These soft-labels, (e.g. integer soft-labels) for unlabeled EMR-data are chosen randomly from a given interval of values. One preferred interval is the interval [0,1] for binary classification.
- Create a semi-supervised model.
  The semi-supervised model ($\theta_{semi}$) is created by maximizing a CPL-function of the Contrastive Pessimistic Likelihood Estimation framework which includes a supervised model for the chosen soft-labels.
- Using the semi-supervised model
  The semi-supervised model ($\theta_{semi}$) is used for updating the randomly chosen soft-labels, in that the CPL-value is maximized.

The steps concerning the CPL-function and the CPL-value are repeated until convergence occurs. Preferably 1000 iterations or more are applied or the steps are preferably repeated until the optimization from one iteration to the next is smaller than 1/1.000.000.

In the following, the basic principle of a CPLE-framework and the above steps are explained. The name Contrastive Pessimistic Likelihood Estimation (CPLE) framework is based on four special aspects of the framework:

"Contrastive": The classifier (i.e. the predicted model) that is trained with labeled data only, is used to estimate the optimization by the not-labeled data. The contrast is the contrast between the supervised model vs. the semi-supervised model that are both in the objective function that is optimized.

Pessimistic: The "objective function" that is maximized during the training, is e.g. here a "Log likelihood" or a "generative likelihood" or a "discriminative likelihood". The soft labels are assigned to the unlabeled data in a way that the improvement of the semi-supervised model with respect to the supervised model is minimal.

Likelihood: The objective function is maximized during the training. The bigger the likelihood, the better the model.

Estimation: The (internal) parameters of the model are estimated.

In an example, the objective Function may be assumed to read:

$$CPL(\theta, \theta_{sup}|X, U) = \min_{q \in \mathcal{M}_{K-1}} CL(\theta, \theta_{sup}|X, U, q) \qquad (1)$$

wherein CPL is the "Contrastive Pessimistic Likelihoods"-function, the objective function, $\theta$ is an unknown semi-supervised model. This model should be found by maximizing the objective function or the parameters for this model for which the function has a maximum, respectively. $\theta_{sup}$ is a supervised model that is trained with labeled data, X stands for features and labels (data) and U stands for unlabeled data (only features). The soft-labels that should be assigned to the non-labeled data are denoted by q (see e.g. "Contrasive Pessimistic Likelihood Estimation for Semi-Supervised Classification", M. Loog, IEEE Transactions on pattern Analysis and Machine Intelligence, vol. 38(3), pp 462-475, 2016).

The function CL of above formula can be written as:

$$CL(\theta, \theta_{sup}|X, U, q) = L(\theta|X, U, q) - L(\theta_{sup}|X, U, q) \qquad (2)$$

wherein L is the log likelihood of the respective model (semi-supervised and supervised, respectively), each dependent on soft-labels q.

The semi-supervised model can then be calculated with the formula:

$$\theta_{semi} = \mathrm{argmax}_{\theta \in \Theta}\, CL(\theta, \theta_{sup}|X, U, q) \qquad (3)$$

A preferred method to accomplish a CPLE-framework comprises the following steps:

a) A supervised model is created, i.e. a model based on labeled data only. This is done by creating a supervised classifier by training and/or tuning based on labeled EMR-data from the EMR-dataset and a predefined grid of hyperparameter values, by using the inner loop of a nested cross validation routine.

b) For data without labels, soft-labels (e.g. integer values) are randomly chosen from a given interval, e.g. the interval [0, 1]. In the case of a binary classification, it is preferred that the values that are >= 0.5 are rounded to label 1, lower values are rounded to label 0. In an exemplary case of a rheumatoid arthritis value '1' would indicate an acute attack in a given time period, value '0' would indicate no attack during this period. This is done by choosing (if needed integer) soft-labels for unlabeled EMR-data randomly from a given interval of values (e.g. [0,1] for binary classification);

c) Creating a semi-supervised model with maximizing the objective function (CPL) for the chosen soft-labels. This is done by creating a semi-supervised model $\theta_{semi}$ by maximizing a CPL-function of the Contrastive Pessimistic Likelihood Estimation framework which includes a supervised model trained on the labeled data.

d) Using the semi-supervised model to adjust the randomly chosen soft-labels so that the CPL-value (from above equation) is maximized. This is done by using the semi-supervised model $\theta_{semi}$ for updating the randomly chosen soft-labels, in that the CPL-value is maximized.

e) The steps c) and d) are repeated (i.e. the steps concerning the CPL-function and the CPL-value) until convergence occurs.

(E) As already stated, the nested cross-validation procedure for model selection is designed for supervised learning algorithms. I.e. in each fold (inner or outer) some model is trained in a supervised manner on a training set and evaluated on a test set. This allows the usage of the labeled data only. In addition to the aspect of the invention of the nested cross-validation to automate the model selection and hyperparameter optimization in modeling the occurrence of clinical events using EMR data, according to another aspect of the invention it is extended to semi-supervised learning based on the CPLE framework described above.

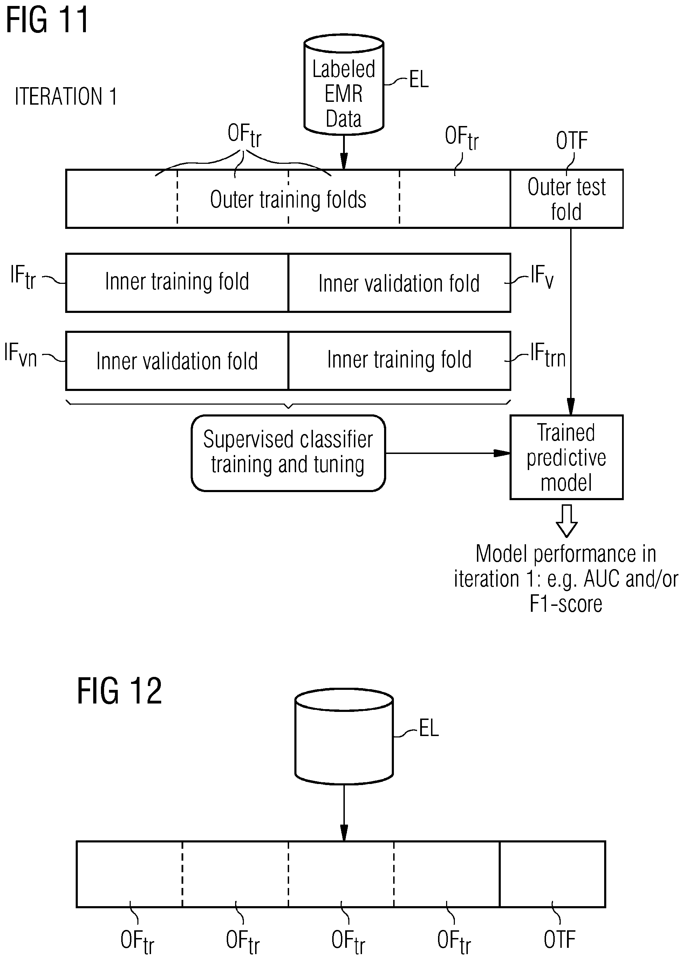

If only labeled data are to be used, a number of supervised learning algorithms are selected including (but not limited to): logistic regression, linear discriminant analysis, quadratic discriminant analysis, decision tree, random forest, adaboost, gradient boost, bagging classifier, k-nearest neighbor and support vector machine. For each algorithm, a grid of values can be created for its most influential hyperparameters, for example various values of the regularization parameter C are defined for the logistic regression as well as type of the penalty function (L1 vs. L2). Then a nested cross-validation procedure can be performed which builds models for each of those algorithms and for each set of their hyperparameters. At the end, the model with the best nested cross-validation performance measure (typically AUC but can also be F1-score, sensitivity, specificity etc.) can be selected as the best one. In this way, the model selection process is automated to a large extent (one still needs to define grid of models and their hyperparameter values manually). If all available EMR data includes the label of interest (e.g. RA flares), then this procedure is sufficient to build and select the best model (note: here under the "best" model it is meant the best within the searched scope of potential models). The first iteration of the procedure is illustrated in Fig. 11. The subsequent iterations are conceptually the same with the exception of the different outer and inner training and test/validation folds which changes in each iteration.

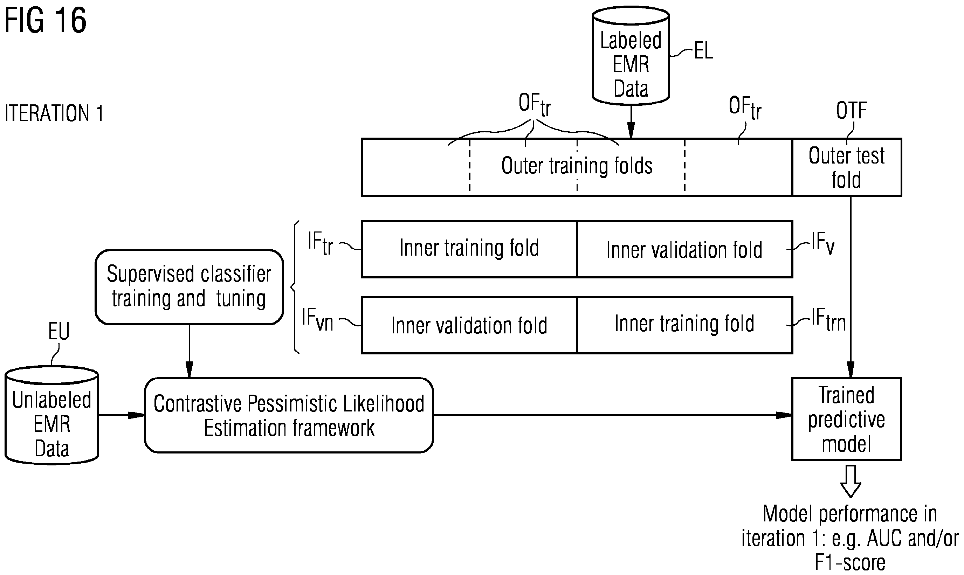

However, as described before, in the EMR data the labels are missing for the large portion of instances. Here we propose the semi-automated model selection procedure based on the nested cross-validation for semi-supervised classification. Building on the solution for problem (D) described above, according to an aspect of the invention the CPLE meta-learner is embedded into the outer loop of the nested cross-validation. When evaluating an algorithm and its hyperparameters, for each fold of the outer nested cross-validation loop a grid search can be performed in the inner loop using labeled training data only to find the best supervised model. Then the CPLE meta-learner is employed receiving the whole dataset (labeled training + unlabeled instances) as well as the best so far obtained model. The CPLE labels the unlabeled instances and retrains the best model, which can then be evaluated on the labeled test set. This procedure is performed k times, once for each of the k iteration of the outer nested cross-validation loop. The results can then be aggregated by computing the mean and standard deviation of the selected performance metrics over all outer loop's iterations. The first iteration of the outer loop in the proposed extension is illustrated in Fig.16. The subsequent iterations are conceptually the same with the exception of the different outer and inner training and test/validation folds which changes in each iteration.

It is important to note that independent of the use of labeled data only or both labeled and unlabeled data, the division to training and test folds is always performed on the group level. The group ID in the EMR data is the patient ID. Such division ensures that all follow-ups (i.e. all data) of a single patient in each of the folds end up either in the training or in the test set. In this way the problem of leakage is avoided, i.e. the model trained in one of the iterations indeed never "saw" any data of patients whose data is used in the test fold of that iteration. In addition to already imposed conservative model performance estimation in the nested cross-validation in comparison to a single cross-validation, this ensures that the estimated performance is further conservative and in the average likely to be slightly better in practice. This is because in the practice, the models will be used to compute predictions for some patients whose past data was used in the model-building process.

For example, each patient has an ID number e.g. 789. All data from this patient collected over years in many follow-ups have the same ID number (789 in this example). The data can be stored in a lot of records, especially for patients with chronic illnesses who need to go to the doctor relatively often. The expression "Group level" refers to this ID number. So every patient represents a group of data. To avoid the phenomenon of "leakage" (which is very negative in machine learning), one should be very cautious when sharing the data in training and testing sets. One has to make sure that all the data in a group (i.e. all patient follow-ups) is converted into either a training set or a test set. Otherwise, if the data is mixed and some patient's FU's in the training set (perhaps future data) and some in the test set, the model already "sees" the patient's future during training. In this case the evaluation of the performance on the test set is not objective. However, by setting this division to two sets at the "group level", one avoids this subtle problem of model accuracy inflation.

In a preferred method, the predictive models are selected from the group comprising logistic regression, linear discriminant analysis, quadratic discriminant analysis, decision tree, extra tree classifier, random forest, adaboost, gradient boost, bagging classifier, k-nearest neighbor, naive Bayes classifier and support vector machine.

In a preferred method, for each iteration of the outer loop of the nested cross-validation a set of hyperparameters relevant for the predictive models the following steps are performed. For the sake of clarity, it is noted that each model could have an individual set of hyperparameters. It is preferred to create a grid of values for a number of the most influential hyperparameters of each predictive model. For example, various values of the regularization parameter C are defined for the logistic regression as well as type of the penalty function (L1 vs. L2).

For each predefined set of hyperparameters a predictive model is trained in the inner loop of the nested cross validation using labeled EMR-data of the current outer loop's iteration, followed by the steps:

- Select the optimal hyperparameter set for the given model.

- Train the inner loop final model using all labeled data of this outer loop's iteration and selected optimal hyperparameters.

- Test the inner loop optimal model using labeled test data of this outer loop's iteration.

- Aggregate test results of the outer loop iterations, preferably by computing the mean and standard deviation of the selected performance metrics over all outer loop's iterations.

- Compare aggregated testing results of the outer loop for different trained predictive models and select the optimal predictive model. The model is selected as the best one with the best nested cross-validation performance measure. Typically, AUC is used, but can also be F1-score, sensitivity, specificity etc.

- Train the final predictive model for a (each) time horizon by repeating iterations of the outer loop for each predictive model using all labeled and especially also all unlabeled data.

In a preferred embodiment, for each outer fold of the outer nested cross-validation loop a grid search is performed in the inner loop using labeled training data only to find the best supervised model.

In a preferred embodiment, the best model is retrained and then evaluated on the labeled test set of the outer fold, wherein this procedure is performed a number of times, once for each of the number of iterations of the outer nested cross-validation loop.

This procedure gives as a result the info, which algorithm and with which hyperparameters are best for the given data. However, in one additional step, the final model is created with all instances (all labeled and preferably all unlabeled instances). In addition, it is also important to find the best model and then train it with all the data for each relevant period.

With this method a prognostic model can be generated that computes the probability of an event (e.g. RA flare) occurring within the next X months of current visit (e.g. the target would be defined due to the time horizon of 3 months). This probability can be visualized at each visit, thus showing the doctors an insight into the changes in the risk of the event dependent on their treatment decisions (e.g. medications and a dosage).

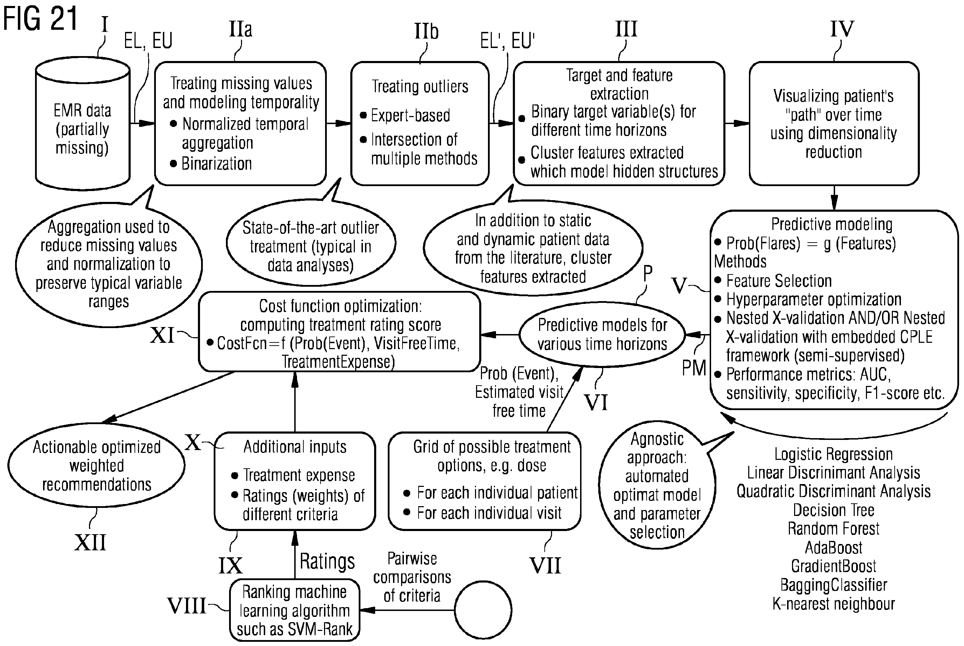

(F) In order to give insights into trade-offs between the risk of an adverse event (e.g. an RA flare, kidney rejection in kidney transplantation, hospital readmission, patient death etc.) over various time horizons (e.g. 1, 3, 6, 9, 12 months from the current follow-up) on the one hand and treatment expenses including indirect expenses caused by frequent patient visits on the other hand, the invention is based according to one of its aspects on an optimization solution explained in the following. This solution relies on all previously stated solutions for the stated problems. Its core is the trained and conservatively evaluated predictive model which predicts probabilities of an adverse event for a certain time horizon. Assuming a treatment decision is made by a clinician (e.g. a medication is selected with a certain dose level), at each follow-up, as the patient data becomes available, it gets provided to the model which predicts an event probability (e.g. death within a year from the current follow-up). Those predictions can be illustrated for all past follow-ups in a graphic such as the one illustrated in Fig. 21.

The results of (F) are preferably achieved by minimizing a cost function.

This solution can be optimized by the following preferred embodiments.

(F1): Several predictive models are generated, one for each time horizon. For physicians, different time horizons are interesting, instead of only one time horizon of e.g. 3 months. Several prognostic models are generated estimating the probability of an event within e.g. one month, three months, or 6, 9 or 12 months. It has to be noted that these time horizons are disease-specific. These models are summarized in a model called a "meta-model". This enables a visualization of the probabilities of the event for several time periods.

(F2): Predictions are calculated with the meta-model not only for the current medication or dose but also for a number or plausible drugs or doses. This provides physicians with insight into how the change in medication and dose will affect the risk of clinical events over time.

(F3): Based on these calculated risks, the time in which no follow-up is necessary can be estimated. That has the advantage that time and resources could be saved. In an example where the likelihood of relapses is low (e.g. < 50%) within the next 1, 3 and 6 months and >= 50% starting from the ninth month, then the physician may make the next visit shortly before the end of the 6th month order. This "visit-free time" could be normalized and quantified on a scale from 0 to 1.

(F4): The costs of medication and dose (which are known to each hospital for each disease) can be normalized (e.g. on a scale from 0 to 1. This has the advantage that the doctor has an overview about the costs, follow-up times and probabilities of the event within different time horizons when there is the need to decide about the appropriate therapy.

(F5): In an example, where physicians have 7 criteria (cost, visit-free time and 5 probabilities of clinical events for 5 time horizons) which they can use to make their decision. However, a recommendation of the decision would be advantageous. To solve this, a number of doctors working in a medical field (e.g. rheumatology) compare the criteria. This could e.g. be accomplished by "pairwise comparisons". Practically this could be a very short survey, in which a doctor would choose the more important criterion between 2 offered criteria (each pair would be presented to a doctor). The results of this survey would be provided to a ranking algorithm (e.g. SVM Rank) and the algorithm generates the aggregated rating for each criterion. These ratings could be normalized on a scale between 0 to 1 and show aggregated weights of the individual decision criteria for the medical field.

(F6): The weights obtained in the previous step are multiplied by the quantitative values of the individual criteria, forming a "Treatment Rating Score". Based on this score, the therapy recommendations are given.

(F7): A Decision Support System, which graphically and transparently presents the information about risks for clinical events over various time periods, especially as well as the need for visits and therapy costs. In addition, the rating of therapy decisions is shown for each patient and the three best decisions are suggested.

A preferred method concerning item (F) for automated clinical decision support for Automated Supervised and Semi-supervised Classification and Treatment Optimization of Clinical Events using EMR Data, comprises the additional step calculating and visualizing the probability of a clinical event for different medication and/or doses with the number of trained prediction models for each time horizon.

A preferred alternative or additional method comprises the step estimating the time when no follow-up is necessary based on the calculation(s) of the probability of a clinical event occurring and preferably obtaining the financial costs for a therapy, especially the financial costs for medication and/or doses and/or a follow-up. The financial costs could be derived only once from an information-source. However, since costs could fluctuate over time, it is advantageous to get the actual estimation about the costs.

A preferred Method for automated clinical decision support for Automated Supervised and Semi-supervised Classification and Treatment Optimization of Clinical Events using EMR Data, comprises the additional steps:

- Computing ratings of included criteria (e.g. treatment cost, visit-free time, probability of a clinical even for different time horizons) using a ranking algorithm (e.g. SVM-Rank) from pairwise comparisons of those criteria as provided by multiple clinicians. This step could especially be performed only once.

- Preferably: Normalize financial costs of various possible treatments (especially medications and/or dosage), preferably in a way that the most expensive treatment (especially medications and/or dosage) correspond to the normalized expense of 1 and the zero dose corresponds to the normalized expense 0. This step could especially be performed only once.

- Preferably: Computing 1-normalized visit-free time, wherein preferably normalized visit-free time is normalized to the interval [0,1] in the following way:

   a) if the event probability is high (e.g. >=50%) already at the shortest relevant time horizon, then the longest "safe" normalized visit-free time is 0 for the given treatment,
   b) if however the predictions for all relevant time horizons are small (e.g. < 50%), the longest visit-free period is 1 for the given treatment.

- Preferably: Computing a Treatment Rating Score for each plausible treatment as a sum of products of computed ratings for relevant criteria and quantitative values of those criteria (e.g. normalized financial costs and 1-normalized visit-free time as well as an event probability in different time horizons).

Preferably, a recommendation for a suitable treatment based on the Treatment Rating Score is evaluated, wherein e.g. treatments with a smaller score are preferred.

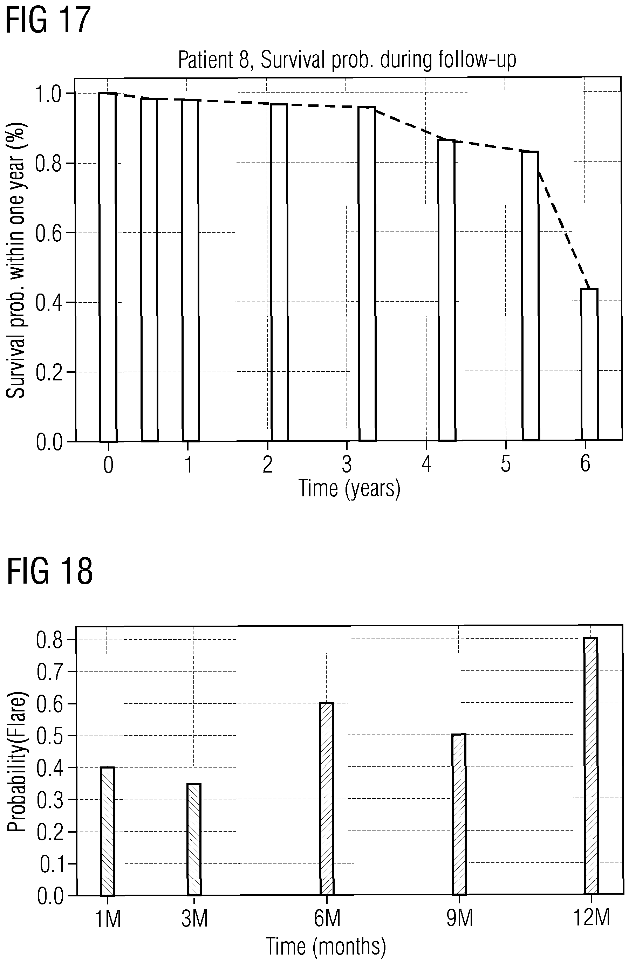

According to a further aspect of the invention, based on the aforementioned system and method for automated semi-supervised classification of disease activity in autoimmune diseases using EMR data, predictive models for an adverse event of interest can be generated for different time horizons (i.e. a meta-model is generated as a collection of multiple predictive models). This is illustrated in Fig. 18 for the RA flare example.

In the following, the presented aspects of the invention are summarized, and additional possible aspects are listed.

According to a further aspect of the invention, missing values in the electronical medical record are determined based one past values using sliding windows with monotonically decreasing weighting functions, in particular using temporal aggregation. The inventor recognized that by using this approach the amount of missing values in numerical variables can be significantly reduced. Furthermore, the temporal context of the measurements is taken into account by giving highest weight to the most recent and lowest weight to the latest relevant value.

According to a further aspect of the invention normalization is based on the summed products of variable values and their corresponding weights. The inventor has recognized that using normalization based on summed products the estimated values are kept in realistic and interpretable scales.

According to a further aspect of the invention a binarization is used to encode the numerical values obtained based on normalized temporal aggregation. The inventor has recognized that in case some missing values remained after performing a normalized temporal aggregation, they can efficiently be modeled as binary vectors using binarization, so that no EMR data gets wasted and every single instance can be used for modeling.

The inventor furthermore recognized that these aspects enable a direct application of the predictive modeling statistical and machine learning algorithms without wasting any EMR data due to the missing values in input variables. Methods according to these aspects of the invention can be used for clinical decision support based on the EMR data. Although motivated by the problems observed in the concrete RA use case, the applicability of the proposed approach is independent of the use case and scales to working with the EMR data in general.

Hidden structures in a highly dimensional EMR data often carry a predictive power that is not explicitly utilized by using the original features only for prediction in classification tasks, such as the task of predicting RA flares. According to a further aspect of the invention the EMR follow-ups are clustered before additional features are derived from clusters (distances to cluster centroids and a cluster label), in particular, these hidden structures can be taken into account explicitly when building classifiers of a disease activity. The inventor recognized that since such features can carry a significant predictive power, the predictive models that use them can have better performance making corresponding prediction algorithms more reliable and accurate.

According to a further aspect of the invention the patient follow-up history including the latest data in the reduced PCA space is animated. The inventor recognized that this animation or visualization enables an easy visualization of disease changes over time and shows a patient's path through a disease. This gives clinicians a clear image and intuition about the disease stability for a given patient over time. Furthermore, since follow-ups associated with an event of interest and those where such event was absent (e.g. RA flare) are statically shown in the background, a clinician can not only see how much the disease activity changes over follow-ups for an individual patient, but also judge if the data collected over time and at the current follow-up are in/closer to the group of "dangerous" or "safe" follow-ups. Based on these proximities, appropriate alarms/warnings can be triggered. Furthermore, such visual assets could prove to be valuable for physicians and would significantly contribute to the acceptance of the digital products based on advanced predictive data analytics.

According to a further aspect of the invention the CPLE framework is applied for labeling the unlabeled EMR data. In particular, for RA flare occurrence within a given time horizon for the follow-ups that lack this information in the model training phase is estimated. The inventor recognized, that first, the CPLE receives a classifier already trained on the labeled data and tries to improve it using the unlabeled data (opposite of the denoising autoencoders, which performs the training on the labeled data afterwards). In the process of training the CPLE, unlabeled instances from the EMR are labeled and used with originally labeled instances together explicitly to make the final model. A supervised classifier applied after training denoising autoencoders in the state of the art is still restricted to using the same (often small) amount of originally labeled data found in the EMR. Furthermore, the original features are preserved, i.e. the patient demographic data, blood laboratory measurements etc. are provided to the supervised classifier (e.g. which predicts the RA flares) and clinicians can see and understand what are its predictions based on and how relevant are certain variables for the prediction. As an additional advantage, if a white-box supervised learning algorithm such as the logistic regression is used, a full insight into the model structure is achieved, i.e. it can be easily seen which EMR variables, how and how much are associated with computed predictions. In contrast, denoising autoencoders provide reduced, abstracted features of the hidden layer to the supervised classifier making it fully black-box and not transparent for clinicians (even if white-box models are used). In particular, these advantages can be used for building more transparent clinical decision support systems, which are likely to have higher acceptancy by clients.

According to another aspect of the invention nested cross-validation is used to automatically evaluate a number of possible machine learning models and their hyperparameters in the task of predicting the occurrence of an adverse event from the EMR data. The restriction is to use the labeled EMR data only. The inventor recognized that by this aspect a large number of possible models for predicting disease activity can be evaluated in an automated manner significantly saving time and resources. Furthermore, no assumptions regarding the model structure and optimal decision boundaries between the classes have to be made, so that the evaluation is done in an agnostic manner selecting the solution which maximizes the classification performance.

According to a further possible aspect of the invention the semi-supervised CPLE learning framework is embedded into the nested cross-validation for using both labeled and unlabeled EMR data. The inventor recognized that automated model selection using also unlabeled data is enable, which typically dominates EMRs, i.e. much larger datasets can be used in the analysis. Furthermore, by relying on the CPLE semi-supervised learning framework, it is unlikely that the solution obtained in this way will be worse than the solution which relies on labeled data only.

According to a further aspect of the invention, the nested cross-validation is capable of automatically detecting if provided EMR data also contains unlabeled instances or not. If this is the case, a method according to Fig. 12 or Fig. 16 will be executed; otherwise a method according to Fig. 11 will be executed. The inventor recognized that when modeling the EMR data, the user will not have to differentiate between labeled and unlabeled data, and that the appropriate procedure will be selected automatically which saves the modeling time and costs.

According to a further aspect of the invention, in possible cases where using labeled data alone provides better predictive performance than adding unlabeled data (such "unusual" cases are known in the literature), the proposed procedure will still select the best model. In particular, modeling with labeled data only vs. using unlabeled data as well is performed automatically and the best model is returned. The inventors recognized that the analyst does not have to perform separate analyses for these two cases and compare the results, which additionally saves time and money.

According to a further aspect of the invention a meta-model is proposed, which comprises of models for predicting a probability of an adverse event for different time horizons. The inventor recognized that by this aspect the user (clinician) gets a deeper insight into the effects of his/her prescribed treatment (e.g. medications and dose) over time, in particular for different time horizons.

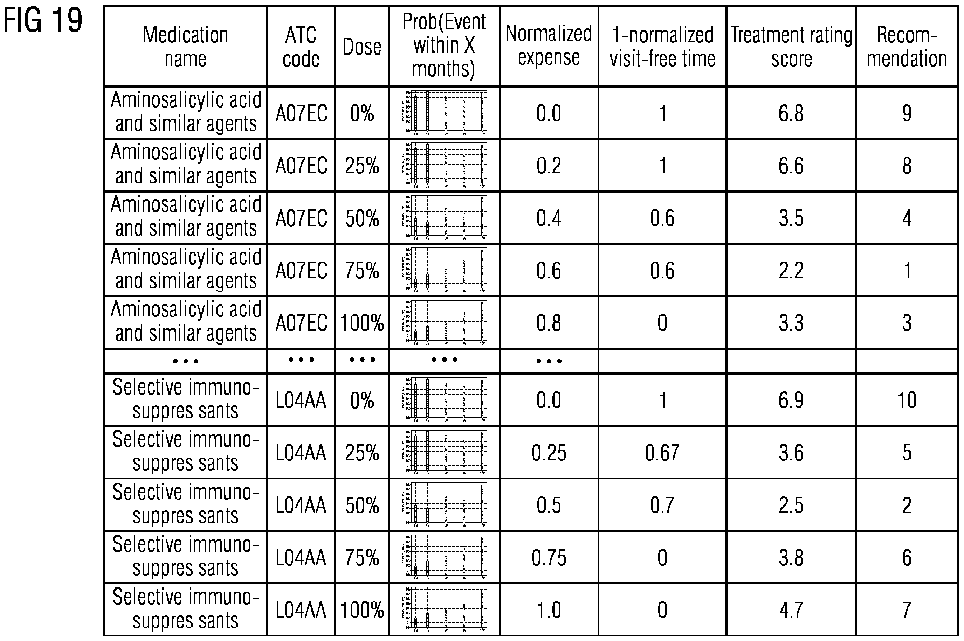

According to a further aspect of the invention, the previous aspect is provided not only for the currently prescribed treatment but also for all feasible treatments. The inventor recognized that a "what if" analysis enables to simulate the meta-model with various possible treatment decisions defined in a grid (e.g. various medications and their dose). In particular, it is possible to provide their overview together with graphically represented effects of the corresponding treatment decisions on the disease activity over different time horizons to the user (as illustrated in Fig. 19, columns 1-4) .

According to a further aspect of the invention a possible visit-free time (i.e. the time in which no follow-up is required since the predicted risk of an adverse event is low) is estimated based on the predicted event probability for different time horizons. The inventor recognized that based on this aspect a clinician can plan patient follow-ups based on the predicted future disease activity and make better schedules saving time.

According to another aspect of the invention, both estimated visit-free time and treatment expense are used for building a treatment recommendation system in addition to estimated event risk for different treatments. In particular, treatment expenses (e.g. monetary cost of drugs) are known to clinicians/their institutions. The inventors recognized that such a recommendation system takes not only different event risks into the account, but also the available resources at the hospital, like clinician's time and available budget.

According to a further aspect of the invention, the computing of treatment recommendations is based on a rating of relevant criteria based on the aggregated feedback (pairwise comparisons) provided by clinicians at the corresponding department. The inventors recognized that by this aspect a uniform rating is established for the whole department for various optimization criteria (e.g. expense vs. event risk for the horizon of X months) using algorithms such as SVM-Rank. In this way, the variation in treatment approaches between different clinicians is modeled and enables better computation of the treatment recommendation score.

According to a further aspect of the invention a treatment recommendation score is calculated taking into account various criteria (different risk probabilities, visit-free time as well as the treatment expense) which are weighted according to their importance to clinicians. The inventor recognized that this score can be used to rank treatment recommendations and propose the best one (or a couple of the best ones) to a clinician.

According to a further aspect of the invention, a clinical decision support system is created in almost an automated manner based on several or all aspects of the presented invention. The inventor recognized that by this aspect the modelers task is simplified and he/she can focus more on understanding the clinical questions and their impact on models and not that much on the modeling itself. Furthermore, the clinicians profit from the transparency of models included in the meta-model as well as from the transparency of the recommendation system.

In the previous parts the invention was described in terms of a method. Furthermore, one aspect of the invention is a data processing system configured for executing the described methods. In particular, the data processing system can comprise a calculation unit, a memory unit and/or an interface.

Such a data processing system (or Computer network system) can, for example, comprise a cloud-computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software.

The data processing system may be a (personal) computer, a workstation, a virtual machine running on host hardware, a microcontroller, or an integrated circuit. As an alternative, the data processing system can be a real or a virtual group of computers (the technical term for a real group of computers is "cluster", the technical term for a virtual group of computers is "cloud").

An interface can be embodies as a hardware interface or as a software interface (e.g. PCI-Bus, USB or Firewire). In general, a calculation unit can comprise hardware elements and software elements, for example a microprocessor, a field programmable gate array (an acronym is "FPGA") or an application specific integrated circuit (an acronym is "ASIC"). A storage unit (another name is "memory unit") can be embodied as non-permanent main memory (e.g. random access memory) or as permanent mass storage (e.g. hard disk, USB stick, SD card, solid state disk). The input/output unit can comprise means for inputting data (e.g. a keyboard or a mouse) and/or means for outputting data (e.g. a display or a printer).

In another aspect the invention relates to a computer program product comprising a computer program, the computer program being loadable into a memory unit of a data processing system, including program code sections to make the data processing system execute a method according to an aspect of the invention when the computer program is executed in said data accessing system.

In another aspect the invention relates to a computer-readable medium, on which program code sections of a computer program are saved, said program code sections being loadable into and/or executable in a data processing system to make the data processing system execute one of the aspects of the invention when the program code sections are executed in the data processing system.

The realization of the invention by a computer program product and/or a computer-readable medium has the advantage that already existing providing systems can be easily adopted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example a documentation or a software key for using the computer program.

In a preferred embodiment, the nested cross validation is not absolutely necessary. This embodiment pertains to using one or more of the following methods (e.g. in combination) for treating EMR-datasets and/or for obtaining results from EMR-datasets:

a) Estimate missing values in the EMR-datasets by using a sliding weighting function, wherein especially missing values are estimated from measurements of the EMR-datasets in a relevancy time window as a sum of weighted past known values, preferably by normalizing this sum by the sum of weights.
And/or

b) Model the temporality of measurements in the EMR-datasets explicitly by value aggregation, wherein in order to fully account for the temporality in the data, known measurements are preferably replaced by the aggregated values.
And/or

c) Form subgroups of related data from different follow-ups within the EMR datasets by using a clustering algorithm,

-   wherein the optimal number of subgroups for a given EMR-dataset is determined,
-   and wherein the subgroups are used as predictors in the classification of clinical events in that for each follow-up
-   a first feature is created from the cluster membership of the follow-up,
-   other features are created from the Minkowski distance of the follow-up to the center of each subgroup.

And/or

d) Reduce the number of data dimensions in the EMR-datasets and the EMR-datasets in reduced number of dimensions are visualized.
And/or

e) Perform a nested cross-validation,

-   wherein for each iteration of the outer loop of the nested cross-validation a set of hyperparameters relevant for the predictive models, wherein it is preferred to create a grid of values for a number of the most influential hyperparameters of each predictive model,
-   wherein for each predefined set of hyperparameters a predictive model is trained in the inner loop of the nested cross-validation using labeled EMR-data of the current outer loop's iteration,
-   followed by the steps:

    -   select the optimal hyperparameter set for the given predictive model,
    -   train the inner loop final model using all labeled EMR-data of this outer loop's iteration and selected optimal hyperparameters,
    -   test the inner loop optimal predictive model using labeled test data of this outer loop's iteration,
    -   aggregate test results of the outer loop iterations, preferably by computing the mean and standard deviation of the selected performance metrics over all outer loop's iterations,
    -   compare aggregated testing results of the outer loop for different trained predictive models and select the optimal predictive model,
    -   train the final predictive model for a time horizon by repeating iterations of the outer loop for each time horizon and each predictive model using all labeled EMR-data and all unlabeled EMR-data.

    And/or

f) In the case that the EMR-datasets comprise unlabeled EMR-data, retrain the inner loop optimal model of the nested cross-validation using a Contrastive Pessimistic Likelihood Estimation framework assigning soft-labels to unlabeled EMR-data, wherein for labeling unlabeled EMR-data of the EMR-datasets the following steps are preferably applied:

- create a Supervised classifier by training and/or tuning based on labeled EMR-data from the EMR-dataset and a predefined grid of hyperparameter values, by using the inner loop of a nested cross validation routine,
- choosing soft-labels for unlabeled EMR-data randomly from a given interval of values,
- create a semi-supervised model $\theta_{semi}$ by maximizing a CPL-function of the Contrastive Pessimistic Likelihood Estimation framework which includes a supervised model for the chosen soft-labels,
- using the semi-supervised model $\theta_{semi}$ for updating the randomly chosen soft-labels, in that the CPL-value is maximized,
- repeating the steps concerning the CPL-function and the CPL-value until convergence occurs.

And/or

g) Calculate and especially visualize the probability of a clinical event in all relevant time horizons, with the number of trained prediction models, especially for different medication and/or doses with the number of trained prediction models for each time horizon.
And/or

h) Estimate the time when no follow-up is necessary based on the calculation(s) of the probability of a clinical event occurring.
And/or

i) Obtain the financial costs for a therapy, especially the financial costs for medication and/or doses and/or a follow-up.
And/or

j) Compute ratings of included criteria using a ranking algorithm from pairwise comparisons of those criteria as provided by multiple clinicians, and

- preferably normalize financial costs of various possible treatments, preferably in a way that the most expensive treatment correspond to the normalized expense of 1 and the zero dose corresponds to the normalized expense 0,
and/or
- preferably compute 1-normalized visit-free time, wherein preferably normalized visit-free time is normalized to the interval [0,1] in the following way:

  a) if the event probability is high already at the shortest relevant time horizon, then the longest "safe" normalized visit-free time is 0 for the given treatment,
  b) if however the predictions for all relevant time horizons are small (e.g. < 50%), the longest visit-free period is 1 for the given treatment,

  and/or
- preferably compute a Treatment Rating Score for each plausible treatment as a sum of products of computed ratings for relevant criteria and quantitative values of those criteria.

Wherever not already described explicitly, individual embodiments, or their individual aspects and features, can be combined or exchanged with one another without limiting or widening the scope of the described invention, whenever such a combination or exchange is meaningful and in the sense of this invention. Especially some features described here could form individual inventions, especially in combination with other features of this description. Advantages which are described with respect to one embodiment of the present invention are, wherever applicable, also advantageous of other embodiments of the present invention.

Other objects and features of the present invention will become apparent from the following detailed descriptions considered in conjunction with the accompanying drawings. It is to be understood, however, that the drawings are designed solely for the purposes of illustration and not as a definition of the limits of the invention.

Fig. 1       displays the binarization approach for categorical and numerical variables.

Fig. 2       displays various weighting functions in a sliding window.

Fig. 3       displays the unsupervised predictive maintenance approach of jet engines based on the PCA.

Fig. 4       displays the k-fold cross-validation for k = 10.

Fig. 5       displays the nested cross-validation. In this example, the outer loop has k=5 folds while the inner loop has
             k=2 folds.

Fig. 6       displays the sliding weighting function for estimating missing values and for accounting for temporality in the
             EMR laboratory measurements.

Fig. 7       displays the binary encoding step performed after normalized temporal aggregation.

Fig. 8       displays the proposed approach for deriving cluster features from the EMR data.

Fig. 9       displays a proposed technical feature.

Fig. 10      displays the CPLE framework of a preferred example.

Fig. 11      displays the first iteration of the outer loop of the nested cross-validation applied to the labeled EMR data.

Fig. 12      displays the outer loop of a nested cross-validation.

Fig. 13      displays the inner loop of a nested cross-validation.

Fig. 14      displays the outer loop of a nested cross-validation for another iteration.

Fig. 15      displays the outer loop of a nested cross-validation for another iteration.

Fig. 16      displays the first iteration of the outer loop of the nested cross-validation which incorporates the CPLE framework for making use of the unlabeled EMR data.

Fig. 17      displays visualized predicted survival probabilities (in general these could be event-free probabilities for a
             custom clinical event such as RA flare or hospital readmission) within a time horizon of one year for a patient,
             given some medication and dose decided by a clinician.

Fig. 18      displays the predicted RA flare probabilities (generalizable to any clinical event such as e.g. death) for an RA
             patient for five different time horizons computed at one patient visit for a given treatment.

Fig. 19      displays recommendation system based on the multiobjective optimization.

Fig. 20      displays the ranking procedure based on the SVM-Rank algorithm.

Fig. 21      displays a system and/or a method for automated semi-supervised classification and treatment optimization
             of clinical events using EMR data in the training phase.

Fig. 22      displays a system and/or a method for automated semi-supervised classification and treatment optimization
             of clinical events using EMR data in the productive or application phase.

Fig. 23      displays a preferred method for creating predictive models for an automated clinical decision support system
             for automated supervised and semi-supervised classification and treatment optimization of clinical events
             using EMR data.

Fig. 24      displays a preferred method for automated clinical decision support for automated supervised and semisupervised classification and treatment optimization of clinical events using EMR data.

Fig. 1 shows the binarization approach for categorical and numerical variables. In view of a measured value,
here e.g. values from measurements of the Creatinine level or the level of leukocytes, and one or more given thresholds
(here two thresholds for creating three ranges: low, normal and high), binary variables are created. A '1' is applied if a
value lies in one of the intervals given by the thresholds, a '0' is applied if not. In the first line of fig. 1 the measured
Creatinine value is higher than the upper threshold, what results in a '1' in the "Creatinine high" column. In the first and second line of fig. 1 the measured leucocytes value is between the upper and lower threshold, what results in a '1' in the "Leukocytes Normal" column.

Fig. 2 shows various weighting functions in a sliding window for modeling temporal dependencies of clinical events. The time frame (the length of the sliding window) defined here is about 90 days. In total there are nine weighting functions defined that differ in their decay over the time. The decay of the weighting functions shows the decrease of the relevancy of the past data for the current disease activity the further the point of measurement lies in the past. All weighting functions having their maximum value of 1 at the most recent follow-up recorded in the patient's EMR and its minimum value of 0 at the last time point (90 days) within the relevancy time frame. Weighting function w1 shows a rapid decrease soon after the recent follow up, so that the contribution of previous follow-ups in the past is neglectable, w9 shows only a minimum decay over a long period, so that that the contribution of previous follow-ups in the past is serious, w5 shows a linear decay so that the contribution of previous follow-ups in the past linearly decreases the more time has been elapsed.

Fig. 3 shows the unsupervised predictive maintenance approach of jet engines based on the PCA. Changes in the health condition of an engine are represented as a path in the PCA space (marked in the upper image). Warnings and alarms are triggered when the path enters the intermediate and the outer zone in the lower picture, respectively.

The PCA space can be received by dimensionality reduce relevant parts of the EMR-data using methods based on Principal Component Analysis (PCA). By uniting corresponding points (representing corresponding measurements of different follow-ups), the static visualization can be used to show changes over time.

Fig. 4 shows the k-fold cross validation for k = 10. In this procedure, the available dataset is divided in 10 mutually exclusive partitions ("folds"), mostly by random sampling without replacement. In the first iteration, 9 folds ("training folds") are used for training a model which is then evaluated on the remaining fold ("test fold"). This is repeated 10 times, each time choosing a different test fold. In each iteration, some performance metrics is computed, such as the Area Under the receiver operating Characteristics (AUC) or classification error. Afterwards, the mean value and the standard deviation of the performance metrics are computed over the 10 iterations.

Fig. 5 shows the nested cross-validation. In this example, the outer loop has k=5 folds while the inner loop has k=2 folds based on the training folds of the outer loop. The inner loop tunes the hyperparameters and the outer loop estimates the performance.

Fig. 6 displays the sliding weighting (linear) function for estimating missing values M (black dot in the figure; Xs represent known values V) and for accounting for temporality in the EMR laboratory (numerical) measurements. In the figure there are two measurements in the relevancy time window W. Thus, a missing value M (dot) can be estimated as the sum of weighted past known values V. In order to fully account for the temporality in the data, known measurements can, thus, be replaced by the aggregated ones.

However, if there are no known values V within the relevancy time frame, the missing value will remain missing, as shown in figure 7.

Fig. 7 displays the binary encoding step performed after normalized temporal aggregation. After performing the normalized temporal aggregation, a binarization approach is applied to encode the remaining missing values as binary zero vectors. This is done e.g. as explained in the example of figure 1 by first creating a dataset of values (in this figure a table with five values) and then binarizing these values by comparing them with given thresholds.

The side effect of performing the steps illustrated in Fig. 6 and Fig. 7 is that predictive algorithms gets structures in a tabular format without missing values and a wide range of statistical and machine learning algorithms for predictive modeling can be applied.

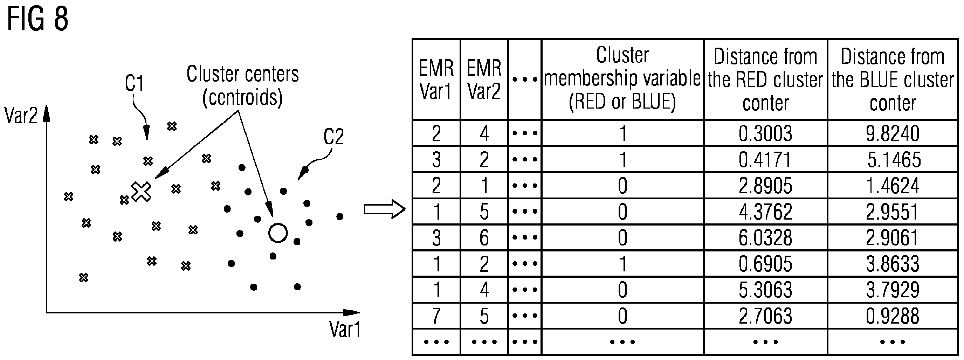

Fig. 8 displays the proposed approach for deriving cluster features from the EMR data. Different clusters C1, C2 are denoted by different symbols in a plane defined by two different parameters Var1, Var2 used for clustering the follow-ups of the EMR-data. Once the clusters C1, C2 of patients' follow-ups are obtained, they are used as predictors in the classification of disease activity (e.g. flare prediction) as described in the following.

For each follow-up the cluster membership becomes one feature (see 4th column of the table) and for each follow-up the Minkowski distance to the center of each cluster (big X and big dot) becomes an additional feature (see columns 5 and 6). In this way, the following number of additional predictors is created: (number of clusters + 1). Here the Clustering-Algorithm has found two subgroups (clusters) in the EMR-datasets. The "additional predictors" are the distances from the center of these two clusters (in this case 2 distances for the two clusters), as well as the "membership" concerning the clusters. Therefore, in this example the number of additional predictors derived is 3, what is number of clusters + 1 for the membership information. In a simple example, the additional predictors are simply additional variables (e.g. columns in a table) with values that could be used for predictions. In the case there are 3 clusters recognized in the EMR-datasets, there are derived 3 distances from 3 centers plus the membership, this are 4 values in total.

In this figure it is assumed that two clusters C1, C2 are optimal for the given dataset. The Figure is simplified for illustration purposes; the approach generalizes to an arbitrary number of EMR variables and optimal clusters C1, C2.

Fig. 9 displays a proposed technical feature. Each dot represents one patient follow-up. The black dots represent follow-ups after which adverse event (e.g. RA flare or even patient death) did not happen within the time horizon of interest (e.g. 3 or 6 months). The white dots are those associated with the occurrence of the adverse event. These are mostly grouped on the right side of the 2D PCA space. The group of light shaded dots on the lower left represent all available follow-ups of a single patient that never experienced an adverse event. One can see that they are all mapped to the left side of the graph, where follow-ups without adverse event are grouped. Moreover, the patient data is stable over multiple follow-ups, i.e. the variance of the PCA values over time is relatively small as the light shaded dots (of this group) fall close to each other. In contrast to this patient, dark shaded dots represent the follow-ups of a patient who eventually did experience an adverse event (last dark shaded dot on the right). The black arrow is added to illustrate the temporal order of those follow-ups (normally, this will be presented to clinicians as animation). The first dark shaded dot on the left (i.e. mapped data of the first follow-up) is relatively deep within the black cluster. Each subsequent follow-up is mapped more to the right, toward the "dangerous" white cluster. Moreover, in contrast to the light shaded dots, the variance of this patient's data is high. Having such (animated) visualization of patient visits can help clinicians to get an impression about the disease activity over time. Warnings and alarms can be triggered when the follow-up data get mapped closer to the "dangerous" cluster of the follow-ups.

Fig. 10 displays the CPLE framework of a preferred example. It uses both labeled EMR-data EL and unlabeled EMR-data EU in the EMR-datasets as well as a supervised classifier trained on the labeled data only. The final trained predictive model expects the inputs in the same form as they are given in the EMR, i.e. no abstract features are created.

The CPLE includes the original supervised learning solution (i.e. a classifier trained on the labeled data only) explicitly into the objective function which is optimized, assigning "soft" labels to the unlabeled data. In this way, the potential improvements of the solution are controlled, i.e. the resulting classifier should not perform worse than the one trained on the labeled data only. The amount of improvement depends on the dataset itself as well as on the ratio of the labeled and unlabeled data. Typically, when the number of labeled instances is large, the inclusion of unlabeled instances rarely brings significant improvements and vice versa. With small adjustments, other semi-supervised algorithms (like transductive support vector machines or self-learning classifiers) can be used as well.

Fig. 11 displays the first iteration of the outer loop of the nested cross-validation applied to the labeled EMR data EL.

The nested cross-validation is explained with the help of figures 12 to 15. The following pseudocode should help to get an overview over the practical process performed by the nested cross validation, wherein the steps 1, 2, 3 and 4 ("For...") are loops surrounding parts of the code. Loop (1) surrounds all following steps, loop (2) surrounds steps (3) to (9), loop (3) surrounds steps (4) to (8) and loop (4) surrounds step (4a).

(1) For each relevant time horizon (e.g. 1 month, 3 months, etc.; for each horizon one final best model will be created):
(2) For each untrained model which can output probabilities (from the predefined set of models e.g. including RandomForest, AdaBoost, Logistic Regression etc.).
(3) For each iteration of the outer loop of the nested cross-validation.
(4) For each predefined set of hyperparameters (defined in a grid) relevant for the given untrained model.
(4a) Train a model in the inner loop of the nested cross validation using labeled training data of the current outer loop's iteration.
(5) Select the optimal hyperparameter set for the given model.
(6) Train the inner loop final model using all labeled data of this outer loop's iteration and selected optimal hyperparameters.
(7) If unlabeled data available: retrain the inner loop optimal model using the CPLE Framework which assigns soft labels to unlabeled data.
(8) Test the inner loop optimal model using labeled test data of this outer loop's iteration.
(9) Aggregate test results of the step (3) over outer loop iterations.
(10) Compare aggregated testing results of the outer loop for different trained models and select the optimal one.
(11) Train the final model for this time horizon by repeating the steps (4) - (7) for the optimal model using all labeled and all unlabeled data.

Figures 12 to 15 show steps (3) to (9) of this pseudocode. In an example it could be assumed that step (1) starts with the time horizon of 1 month and in step (2) the first model to evaluate is chosen to be Log.-Regression. Now is the task of the steps (3-9) to evaluate various hyperparameter values of Log.-Regression and find the best one (done in the inner loop) and to evaluate the expected performance of the Log.-Regression models trained with optimal hyperparameter values and evaluated on the independent test sets (done in the outer loop) .

Iteration 1 of the outer cross-validation

In Fig. 12, step (3) is shown. In the first iteration of the outer cross-validation all labeled EMR data EL are taken and divided into outer training folds $OF_{tr}$ and one outer test fold OTF (or training and test set of data). The expression "outer" indicates that the folds are situated in the outer loop of the nested cross validation.

Only data from outer training folds $OF_{tr}$ go into the inner loop of the cross-validation. The Outer test fold OTF is kept as an independent test set for later testing. Outer test data is used later in the step (8).

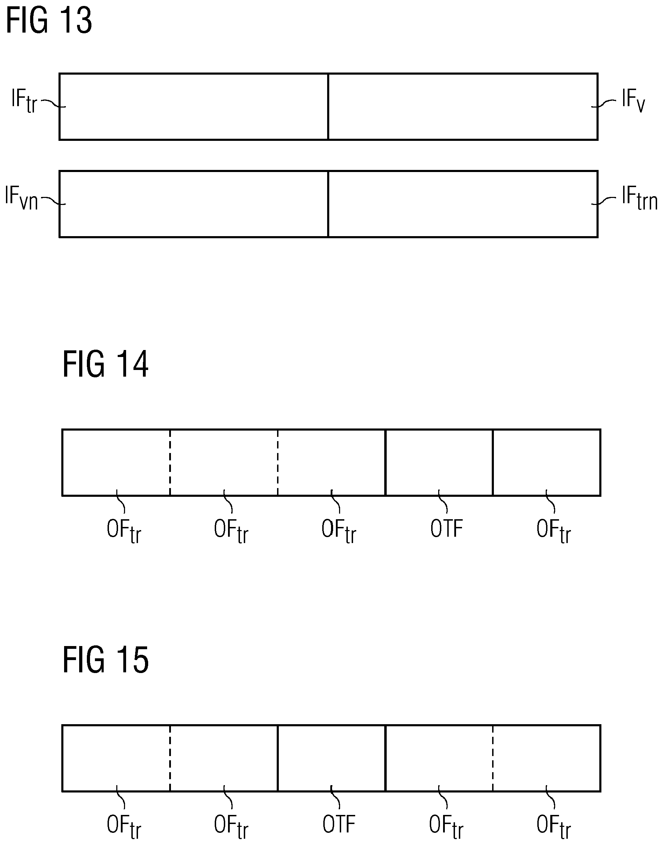

In figure 13, step (4) is shown. For the untrained model that we currently want to evaluate (logistic regression as defined in step (2) and time horizon of 1 months as defined in step (1)), a grid of this model's specific hyperparameters is defined (in the case of logistic regression these are the type of regularization (L1 vs. L2) and the value of the regularization parameter C). These are specific for logistic regression, i.e. when some other model will be evaluated e.g. random forest, there will be some other hyperparameters, specific for this other model.

| Untrained model | type of regularization | C value |
|---|---|---|
| Log.-Regression | L1 | 0.01 |
| Log.-Regression | L1 | 0.1 |
| Log.-Regression | L2 | 0.01 |
| Log.-Regression | L2 | 0.1 |

This is the so-called grid of hyperparameters for logistic regression. There may be many more values of C that the modeler (data analyst) wants to investigate but normally a couple of "typical" values are taken due to time constrains. Now the step (4) says that for each set of hyperparameters (each of the 4 rows in the table above), a logistic regression model will be "assessed" in the inner cross-validation. It should be kept in mind that the inner cross-validation may use only the data from the training folds of the outer cross-validation.

Step (4a) executes this assessment - in each iteration of the inner cross-validation a model is trained on the training set and tested on the test set (of the corresponding inner loop iteration). The figure below shows how 2-fold inner cross validation (there may be more iterations than just 2, this is decided by modeler/data scientist; the more folds, the longer the procedure will execute. In practice one normally takes 5 or 10 iterations).

In the upper left box, a Log.-Regression model with L1 and C=0.01 is trained on this inner training fold $IF_{tr}$ (data). In the upper right box, a Log.-Regression model with L1 and C=0.01 is validated (tested) on this inner validation fold $IF_v$. Performance of the model is saved for later aggregation.

In the lower right box, again a Log.-Regression model with L1 and C=0.01 is trained but this time on this new inner training fold $IF_{trn}$(data). This new Log.-Regression model with L1 and C=0.01 is validated (tested) on this new inner validation fold $IF_{vn}$. Performance of the new model on this new test data is saved for later aggregation.

After all iterations of the inner loop (i.e. inner cross-validation) are performed, the test results are averaged. Usually a performance metric like accuracy of classification error is logged for each test set and averaged at the end. The output of the step (4a) looks like this:

| Untrained model | type of regularization | C value | Average classification error in % |
|---|---|---|---|
| Log.-Regression | L1 | 0.01 | 30 |
| Log.-Regression | L1 | 0.1 | 20 |
| Log.-Regression | L2 | 0.01 | 10 |
| Log.-Regression | L2 | 0.1 | 20 |

Step (5) does the following:

| Untrained model | type of regularization | C value | Average classification error in % |
|---|---|---|---|
| Log.-Regression | L1 | 0.01 | 30 |
| Log.-Regression | L1 | 0.1 | 20 |
| Log.-Regression | L2 | 0.01 | 10 |
| Log.-Regression | L2 | 0.1 | 20 |

So in the step (5), the set of hyperparameters for logistic regression model which minimizes the classification error is selected as the optimal parameter set. This is the set with L2 and C=0.01 which had the classification error of 10% (third line).

It should be noted, that the interest is in how this logistic regression model with the optimal values of hyperparameters selected in this way would perform in reality on never-seen data. Its error has in fact been measured in the inner loop (10%), but this error is used for selecting the best hyperparameters and that makes it not suitable for making statements about the expected performance in new, never-seen data. That is the purpose of the outer loop: it always keeps a test set which is independent and not used for selecting the hyperparameters. There will be a later use of this set to measure the expected error in the real-world.

Step (6) now takes all the data of the inner cross-validation (i.e. the training data of the corresponding outer loop iteration) and the optimal hyperparameters (L2 and C=0.01) and trains a logistic regression model on this data using the optimal hyperparameter values.

Step (7) checks if unlabeled data is available and if yes, retrains the model from step (6) using the CPLE framework. The output of the step (7) is a logistic regression model with hyperparameters L2 and C=0.01 trained on all the training data of the corresponding outer loop iteration (all are labeled) + all unlabeled data.

Step (8) finally takes the test set of this iteration of the outer loop (all data in this set are labeled) and measured the performance of the model from step (7) on this test set. The result of the step (8) is performance metric value saved for later averaging of the outer loop results (this metric is e.g. classification error or accuracy, or something called AUC or F1-score etc. - any metric suitable for classification problems can be used and this is decided by a modeler. In real projects AUC or F1-score are preferred as they are much more robust than classification error, but in this example for an easier understanding will include classification error) .

By now, there has been estimated the error of the logistic regression model trained using outer training data of the first iteration with the optimal set of hyperparameters found in the inner loop and adjusted/retrained using CPLE and unlabeled data. This performance metric value (e.g. classification error) is the output of the first iteration of the outer loop. As an example this error could be 12%.

Iteration 2 of the outer cross-validation

Now it is proceeded with the second iteration of the outer cross-validation: step (3) is started again, this time with new outer training and test set as illustrated in figure 14.

Steps (4-8) are performed just as described above, and the output of this second iteration of the outer cross-validation is another performance metric value, e.g. 8%.

Iteration 3 of the outer cross-validation

Now it is proceeded with the third iteration of the outer cross-validation: step (3) is started again, this time with new outer training and test set as illustrated in figure 15.

For this new data division, again steps (4-8) are repeated and some estimate of the classification error is achieved, e.g. 15%.

In this illustration, an outer cross-validation with 5 iterations is presented (you already got the idea). In practice, one normally takes either 5 or 10 iterations. The more iterations (this also applies to the inner loop), the longer the execution time. A modeler must decide what is feasible number of iterations for both inner and outer cross-validation in each concrete project.

Iterations 4 and 5 are executed as well and measured the classification errors of e.g. 13% and 6%, respectively.

Step (9) averages these performance metric values over different folds:

| ITERATION of the outer loop | classification error in % |
|---|---|
| 1 | 12 |
| 2 | 8 |
| 3 | 15 |
| 4 | 13 |
| 5 | 6 |

Average error is then (12+8+15+13+6)/5 = 10.8%

Now the whole procedure described above is repeated for the next untrained model (from the list in step (3)). E.g. that model is Random forest. Then in the next iteration of the step (2), next model is evaluated, e.g. adaboost etc. until all the models are evaluated.

In step (10), the model with the smallest aggregated error is selected as the final model for the time horizon given in step (1):

| Model | average error in % |
|---|---|
| log. reg. | 10.8 |
| random forest | e.g. 7 |
| adaboost | e.g. 15 |

So the best model for the evaluated time horizon in this example is random forest as it has the smallest error (7%). Please recall that there were 5 iterations of the outer loop and each iteration produced one random forest model on the corresponding outer training set and evaluated that model on the outer test set. The question now is, which of these 5 models should be used in practice, e.g. deployed at the customer site.

The answer is step (11): a new final random forest model will be trained using all labeled data and all unlabeled data. This can be illustrated as shown in figure 16.

This corresponds to doing steps (4-7) using all the available data. There is no outer loop anymore - it was used just to tell us which model was the best for the given time horizon (in our example this was random forest). This will generate final random forest model for the given time horizon.

Then it is returned to step (1), the next time horizon relevant for the disease (e.g. 3 months) is selected and started with step (2) again to get the best final model for this new time horizon. Maybe this will be adaboost, maybe k-NN classifier - steps (2-10) will discover which one it is and then in step (11) a final model will be trained using all labeled and unlabeled data for the 3 months time horizon.

Then the same will be done for the next time horizon.

Fig. 16 displays the first iteration of the outer loop of the nested cross-validation which incorporates the CPLE framework for making use of the unlabeled EMR data EU. However, as described before, in the EMR-datasets the labels are missing for the large portion of instances. Nevertheless, these EMR-validation datasets could also be used for the nested cross-validation (see above explained step (7). This step (7) is based on the solution to problem (D) above.

As can be seen, the CPLE meta learner is embedded inside of the outer loop of the nested cross-validation. When evaluating an algorithm and its hyperparameters, for each fold of the outer nested cross-validation loop a grid search can be performed in the inner loop using labeled training data only to find the best supervised model. Then the CPLE meta learner is employed receiving the whole dataset: unlabeled EMR-data, labeled EMR-data EL training and the best so far obtained model (by a supervised classifier training and tuning). The CPLE labels the unlabeled instances and retrains the best model, which can then be evaluated on the labeled test set. This procedure is performed once for each of the iterations of the outer nested cross-validation loop. The results can then be aggregated by computing the mean and standard deviation of the selected performance metrics over all outer loop's iterations. Here, the first iteration of the outer loop in the proposed extension is illustrated in Fig. 16. The subsequent iterations are conceptually the same with the exception of the different outer and inner training and test/validation folds which changes in each iteration.

Fig. 17 displays visualized predicted survival probabilities (in general this can be any clinical event-free probability, not just death-free) within a time horizon of one year for a patient, given some medication and dose decided by a clinician. In this figure one can see that at the first seven follow-ups (first seven bars) the predicted survival probability within a year from the current follow-up was pretty high (about 80% to 100%). However, at the last follow-up at the 6th year of treatment (eighth bar), this probability dropped to under 50% and this patient actually died a couple of months after the last follow-up. If the clinician had such model and visualization of its predictions, he/she could have experimented with different treatments in order to try to prevent the adverse event.

Fig. 18 displays the predicted RA flare probabilities (in general this can be any clinical event probability) for an RA patient for five different time horizons computed at one patient visit for a given treatment. These predictions are computed by five different machine learning models which form a meta-model.

In this figure, the first two bars (shaded from left to right) illustrate predicted flare probability lower than 50% while the last three bars (shaded from right to left) mark flare probability higher or equal to 50% (this threshold can be decided by clinicians). Predictions are computed for five time horizons of interest (domain dependent; these time horizons might be relevant for the RA disease but can look differently for some other disease). So the first bar shows about 40% probability that the patient will experience a flare within 1 month from the current visit. The second bar shows about 35% probability that the patient will have a flare within 3 months from the current visit (with the same treatment as in the first time horizon of 1 month). The third bar shows probability over 50% that the patient will have a flare within 6 months from the current visit and so on. Such insight enables the clinician to evaluate how long the "flare-free" period would last for a given patient and a given treatment decision. Using this information, it can be estimated when the next patient visit would be necessary. For the example in Fig. 18, the clinician could order the next examination shortly before three months from the current visit expire because the predictive models for both 1 month and 3 months time horizons predict relatively low probability of the adverse event.

In addition to visualizing adverse event probabilities over past follow-ups of a single patient (Fig. 17) and computing and visualizing predictions for different time horizons (Fig. 18). The simulation strategy could be employed for various treatments (in RA case these are medications and dose) to compute adverse event probabilities for all prediction horizons of interest. This is illustrated in Fig. 19.

Fig. 19 displays recommendation system based on the multi-objective optimization. Inputs to the optimization problem are weighted event probabilities for various time horizons, follow-up necessity, as well as the monetary treatment expense. In this figure, the system rated clinician's options and may mark the three most promising ones with are the columns 4, 5 and the third from below in color.

The first three columns in Fig. 19 relate to the treatment (in this illustration the RA example is given). Here different medications and possible dosage options are listed. Since these represent an input to predictive models for different time horizons, probabilities of an adverse event (e.g. RA flare) are computed and graphically illustrated (column 4). Ideally, for all time horizons event probabilities should be minimized. Normalized expense of the treatment is shown in column 5. Normalization is done in a way that the most expensive drug and dosage correspond to the normalized expense of 1 and the zero dose corresponds to the normalized expense 0. Of course, one would want to minimize this criterion.

The column 6 relates to the normalized latest next follow-up. Depending on the predicted event probability, longest "safe" visit-free time is defined. The larger this time is, the better, i.e. it should be maximized if possible. The visit-free time is normalized to the interval [0,1] in the following way: if the event probability is high (e.g. >=50%, but this threshold can be different, depending on the application area of the proposed solution) already at the shortest time horizon of 1 month, then the longest "safe" normalized visit-free time is 0 for the given treatment. If however the predictions for all relevant time horizons are small (e.g. < 50%), the longest visit-free period is 1 for the given treatment. Since this criterion is supposed to be maximized, we transform it into the minimization problem for consistency with other criteria by computing "1-normalized visit-free time", which is given in column 6 in Fig. 19.

In the example above, there are five relevant predicted probabilities (in other examples or diseases there might be more or less of them), normalized monetary treatment expense and 1-normalized visit-free time as criteria that should be minimized in an optimization problem. As stated in point 1, different clinicians treat their patients in different ways. Some may be more liberal while the others may be more conservative. This means that different aforementioned criteria will have different weights/importance to different clinicians, i.e. some would weight risk of flaring within 3 months higher than the necessary follow-up frequency and for the others the budget also plays a very important role.

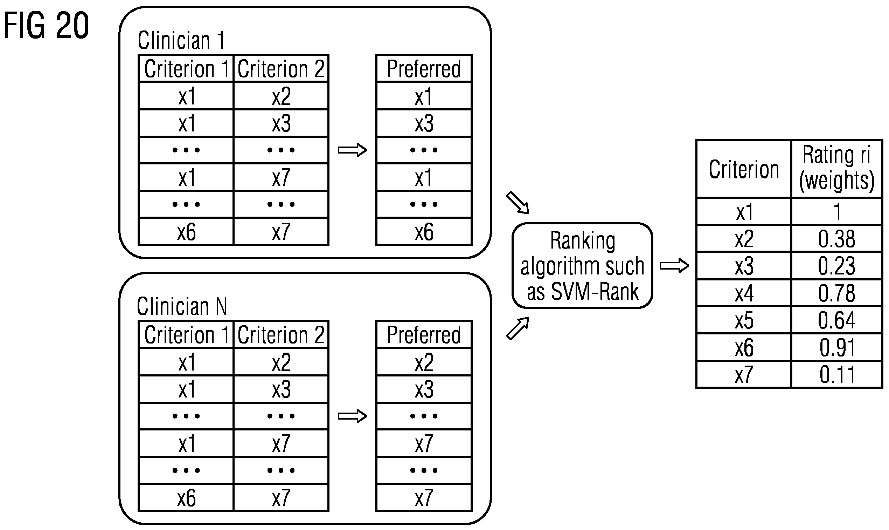

In order to offer common treatment recommendations which summarize approaches of different clinicians and take into account all 7 criteria, according to one of its aspects the invention has the technical features: Each clinician in a department who treats patients (e.g. rheumatology department) performs pairwise comparisons of these criteria marking the one which is in their opinion more important. For 7 criteria, there are 7 x 6 / 2 = 21 pairwise comparison that need to be made. Assuming one needs about 10 seconds to decide which of the offered two criteria is more important to him/her, this procedure would take about 210s = 3.5 minutes per clinician. This is not a lot of time and should be feasible for every clinician to accomplish in a timely manner. In particular, a machine learning ranking algorithm such as the SVM-Rank (see e.g. "Training Linear SVMs in Linear Time"; T. Joachims, KDD '06, Aug. 20-23) can be used which generates ratings from pairwise comparisons. This is illustrated in Fig. 20.

Fig. 20 displays the ranking procedure based on the SVM-Rank algorithm. N clinicians perform pairwise comparisons of the seven criteria (in other example / diseases there could be other, less or even more criteria). Clinicians' preferences are input to the algorithm which generates a rating of criteria. These ratings are used as weights in the optimization function to rank recommendations in the clinical decision support system.

Each of the seven criteria of above example would be assigned a rating (i.e. weight) based on the overall assessment of the criteria performed by clinicians. These weights are then normalized to the interval [0,1] where 1 is assigned to the most and 0 to the least important one.

Now the following technical feature can be defined as an optimization problem with the following inputs:

x1: probability of an event within one month
x2: probability of an event within three months
x3: probability of an event within six months
x4: probability of an event within nine months
x5: probability of an event within twelve months
x6: 1 - normalized visit free time
x7: normalized expenses

and an optimization function to be minimized given as:

$$f(x_1, x_2, ..., x_7) = \sum_{k=1}^{7} r_i x_i \qquad (4)$$

Where $r_i$ are weights obtained from the ranking algorithm and pairwise comparisons of the criteria.

For each treatment option that clinician can take, a treatment rating score (column 7 in Fig. 19) that is computed as a result of the function given above. Based on this score, a recommendation is derived (column 8) where three best recommendations are highlighted.

Finally, all aforementioned technical features and methods taken together represent a basis of the system for automated semi-supervised classification and treatment optimization of disease activity in autoimmune diseases using EMR data. This training phase of such system is illustrated in Fig. 21.

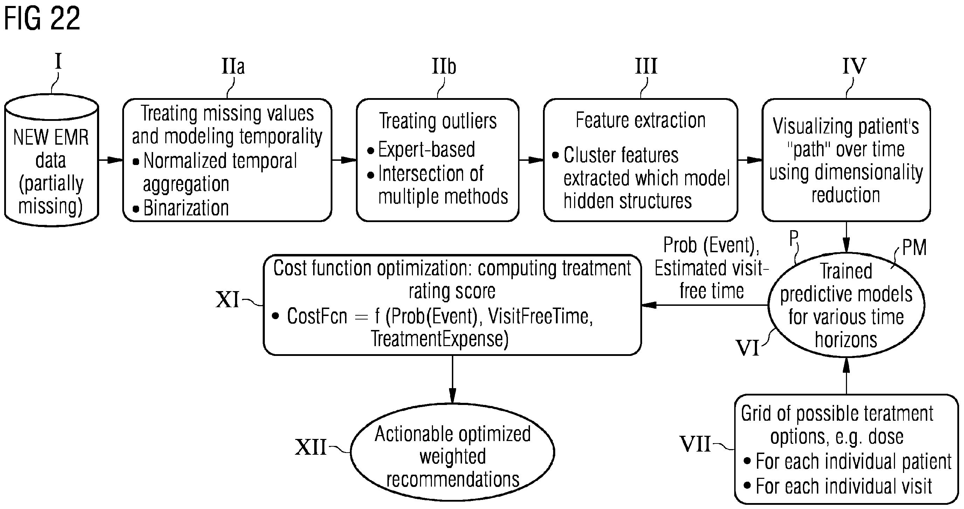

Once trained, the system would be deployed and applied in practice (productive phase). The components of the solution used in the productive phase are illustrated in Fig. 22.

Fig. 21 displays a system and/or a method for automated semi-supervised classification and treatment optimization of disease activity in autoimmune diseases using EMR datasets in the training phase.

In step I, a number of EMR-datasets comprising measurements and patient related data of a number of follow-ups are provided. In this example, some data are partially missing and there could be the case that EMR-data is not labeled (and thus not suitable for a nested cross validation concerning the state of the art). Thus, it is assumed that labeled EMR-data EL and unlabeled EMR-data EU is applied.

Step II, where there is performed a treating of the EMR-datasets in order to estimate missing values and/or to correct outliers and/or to model temporality of measurements, is here divided in two sub-steps IIa and IIb.

Step IIa comprises the action of Treating missing values and modeling temporality. This may include a normalized temporal aggregation and/or a binarization. The aggregation is used to reduce missing values. The normalization is used to preserve typical variable ranges.

Step IIb comprises the action of treating outliers. This could be e.g. a state-of-the-art outlier treatment that is typical analysis. This treatment should be expert-based and/or should comprise a intersection of multiple methods.

Now, the labeled EMR-data EL' and the unlabeled EMR-data EU' is adjusted and ready to be used by the nested cross-validation.

Step III comprises a target and feature extraction. Here two steps of the inventive method, i.e. forming subgroups of related follow ups within the EMR-datasets and providing a target-variable or extract a target-variable from the EMR-dataset, are performed. The target and feature extraction should include a binary target variable (or more) for different time horizons. The forming of subgroups should include the action of clustering features extracted with model hidden structures, e.g. in addition to static and dynamic patient data from the literature, cluster features extracted.

In step IV the patient's "path" over time is visualized using dimensionality reduction. this can be achieved in that the number of data dimensions in the EMR-datasets is reduced and the EMR-datasets in reduced number of dimensions are visualized. The number of data dimensions in the EMR-datasets is preferably reduced by using a Principal Component Analysis ("PCA"), wherein patient data of the EMR-datasets are especially represented in a PCA-space using two or three dimensions and wherein data from follow-ups that are associated with a clinical event are preferably represented in the PCA-space as points of one individual subgroup,
wherein the subgroups are preferably visualized, especially in a space, where dangerous areas are marked.

Step V comprises predictive modeling. Here e.g. the probability of flares is estimated from the features (Prob(Flares)=g(Features)). This could be achieved with feature selection, hyperparameter optimization, nested X-validation (labeled data) and/or nested X-validation with embedded CPLE-framework (semi-supervised, non-labeled data), with the performance metrics: AUC, sensitivity, specificity, F1-score, etc.

This step comprises providing a number of untrained predictive models PM (e.g. logistic regression, linear discriminant analysis, quadratic discriminant analysis, decision tree, extra tree classifier, random forest, adaboost, gradient boost, bagging classifier, k-nearest neighbor, naive Bayes classifier and support vector machine), which can output probabilities and assign weights to EMR-data, wherein each model is capable of being trained with data using methods of machine learning, providing a number of different time horizons, and performing a nested cross validation for each time horizon and for each predictive model PM. Here it is advantageous to apply an agnostic approach, an automated optimal model and parameter selection.

In step VI there is a number of predictive models PM for various time horizons that are readily trained.

To produce a result that can easily be understood by a user (and may comprise other valuable information), in step VII a grid of possible treatment options (e.g. dose) is added to the models. It is advantageous that this grid is created for each individual patient and/or for each individual visit.

In step VIII, a group of physicians (symbol in the circle) perform pairwise comparisons of criteria. The result of these comparisons is generated by a ranking machine algorithm such as SVM-Rank.

The ratings are given to a Routine of Additional Inputs. These additional inputs may be a value for treatment expenses (step X) or ratings (weights) of different criteria (Step IX) that are provided for a final generation of results.

In step XI the final result is generated by minimizing a cost function optimization: a computing treatment rating score. e.g. the probability of an event (Prob(event)), a value for the visit free time (VisitFreeTime), and expenses for a treatment (TreatmentExpense) could be part of this cost function (CostFcn):
CostFcn = f (Prob(event), VisitFreeTime, TreatmentExpense).

In step XII the result of this minimization are provided for a user in form of actionable optimized weighted recommendations.

Fig. 22 displays a system and/or a method for automated semi-supervised classification and treatment optimization of disease activity in autoimmune diseases using EMR data in the productive or application phase. Here Steps I to IV are performed like in above example, however not on old EMR-data, only but at least on an actual EMR-dataset (of a recent follow up) in order to treat the dataset the "right" way so that the trained predictive models are able to "understand" the data of the EMR-dataset.

In step VI there is a number of predictive models for various time horizons that are already trained (e.g. by a method as shown in figure 21).

To produce a result that can easily be understood by a user (and may comprise other valuable information), in step VII a grid of possible treatment options (e.g. dose) is added to the models. It is advantageous that this grid is created for each individual patient and/or for each individual visit.

In step XI the final result is generated by minimizing a cost function optimization: a computing treatment rating score. e.g. the probability of an event (Prob(event)), a value for the visit free time (VisitFreeTime), and expenses for a treatment (TreatmentExpense) could be part of this cost function (CostFcn):

CostFcn = f (Prob(event), VisitFreeTime, TreatmentExpense).

In step XII the result of this minimization are provided for a user in form of actionable optimized weighted recommendations.

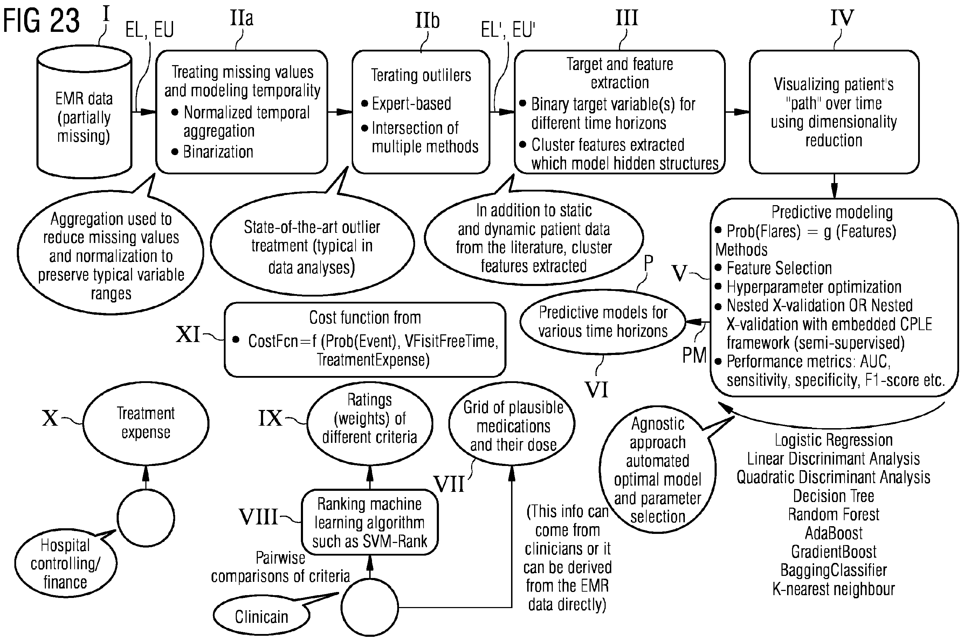

Fig. 23 displays a preferred method for creating predictive models for an automated clinical decision support system for automated supervised and semi-supervised classification and treatment optimization of clinical events using EMR data.

In step I, a number of EMR-datasets comprising measurements and patient related data of a number of follow-ups are provided. In this example, some data are partially missing and there could be the case that EMR-data is not labeled (and thus not suitable for a nested cross validation concerning the state of the art). Thus, it is assumed that labeled EMR-data EL and unlabeled EMR-data EU is applied.

Step II, where there is performed a treating of the EMR-datasets in order to estimate missing values and/or to correct outliers and/or to model temporality of measurements, is here divided in two sub-steps IIa and IIb.

Step IIa comprises the action of treating missing values and modeling temporality. This may include a normalized temporal aggregation and/or a binarization. The aggregation is used to reduce missing values. The normalization is used to preserve typical variable ranges.

Step IIb comprises the action of treating outliers. This could be e.g. a state-of-the-art outlier treatment that is typical analysis. This treatment should be expert-based and/or should comprise a intersection of multiple methods.

Now, the labeled EMR-data EL' and the unlabeled EMR-data EU' is adjusted and ready to be used by the nested cross validation.

Step III comprises a target and feature extraction. Here two steps of the inventive method, i.e. forming subgroups of related follow ups within the EMR-datasets and providing a target-variable or extract a target-variable from the EMR-dataset, are performed. The target and feature extraction should include a binary target variable (or more) for different time horizons. The forming of subgroups should include the action of clustering features extracted with model hidden structures, e.g. in addition to static and dynamic patient data from the literature, cluster features extracted.

In step IV the patient's "path" over time is visualized using dimensionality reduction. This can be achieved in that the number of data dimensions in the EMR-datasets is reduced and the EMR-datasets in reduced number of dimensions are visualized. The number of data dimensions in the EMR-datasets is preferably reduced by using a Principal Component Analysis ("PCA"), wherein patient data of the EMR-datasets are especially represented in a PCA-space using two or three dimensions and wherein data from follow-ups that are associated with a clinical event are preferably represented in the PCA-space as points of one individual subgroup,

wherein the subgroups are preferably visualized, especially in a space, where dangerous areas are marked.

Step V comprises predictive modeling. Here e.g. the probability of flares is estimated from the features (Prob(Flares)=g(Features)). This could be achieved with feature selection, hyperparameter optimization, nested X-validation (labeled data) and/or nested X-validation with embedded CPLE-framework (semi-supervised, non-labeled data), with the performance metrics: AUC, sensitivity, specificity, F1-score, etc.

This step comprises providing a number of untrained predictive models (e.g. logistic regression, linear discriminant analysis, quadratic discriminant analysis, decision tree, extra tree classifier, random forest, adaboost, gradient boost, bagging classifier, k-nearest neighbor, naive Bayes classifier and support vector machine), which can output probabilities and assign weights to EMR-data, wherein each model is capable of being trained with data using methods of machine learning, providing a number of different time horizons, and performing a nested cross validation for each time horizon and for each predictive model. Here it is advantageous to apply an agnostic approach, an automated optimal model and parameter selection.

In step VI there is a number of predictive models PM for various time horizons that are readily trained. If the circle of step VI would show a hardware unit, this would be a preferred prediction-unit P of the invention.

To produce a result that can easily be understood by a user (and may comprise other valuable information), in step VII a grid of plausible medications and their dose is added to the models. It is advantageous that this grid is created for each individual patient and/or for each individual visit. The information for the grid can come from clinicians (lowest round symbol) or it can be derived from the EMR-datasets directly.

In step VIII, a group of physicians (symbol in the circle) perform pairwise comparisons of criteria. The result of these comparisons is generated by a ranking machine algorithm such as SVM-Rank.

In Step IX, the ratings (weights) of different criteria are provided for a final generation of results.

In step X, values for treatment expenses are provided for a final generation of results e.g. by the board of hospital controlling/finance (left round symbol).

In step XI the final result is generated by minimizing a cost function optimization: a computing treatment rating score. e.g. the probability of an event (Prob(event)), a value for the visit free time (VisitFreeTime), and expenses for a treatment (TreatmentExpense) could be part of this cost function (CostFcn):
CostFcn = f (Prob(event), VisitFreeTime, TreatmentExpense).

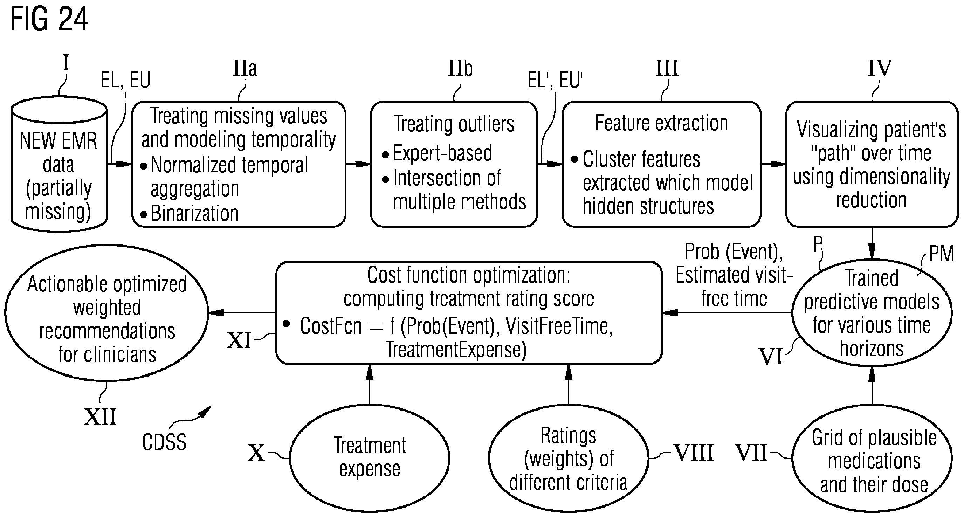

Fig. 24 displays a preferred method for automated clinical decision support for automated supervised and semi-supervised classification and treatment optimization of clinical events using EMR data. If the steps would be units performing these steps, this figure could also show a Clinical Decision Support System (CDSS).

Here Steps I to IV are performed like in above example, however not on old EMR-data, only but at least on an actual EMR-dataset (of a recent follow up) in order to treat the dataset the "right" way so that the trained predictive models are able to "understand" the data of the EMR-dataset.

In step VI there is a number of predictive models for various time horizons that are already trained (e.g. by a method as shown in figure 23).

In Step IX, the ratings (weights) of different criteria are provided for a final generation of results.

In step X, values for treatment expenses are provided for a final generation of results.

In step XI the final result is generated by minimizing a cost function optimization: a computing treatment rating score. e.g. the probability of an event (Prob(event)), a value for the visit free time (VisitFreeTime), and expenses for a treatment (TreatmentExpense) could be part of this cost function (CostFcn):
CostFcn = f (Prob(event), VisitFreeTime, TreatmentExpense).

In step XII the result of this minimization are provided for a user in form of actionable optimized weighted recommendations for clinicians.

In the diagrams, like numbers refer to like objects throughout. Objects in the diagrams are not necessarily drawn to scale.

Fig. 23 shows the training-phase and the collection of necessary information (what are the therapies cost, which drugs and which dose can be used theoretically and which criteria are more important to the doctors than the others). In this figure, there are four results that will be used when using the system, namely the meta-model (see step VI, comprising several prognostic models for different time periods), the ratings of the criteria in pairwise comparisons (see step IX), theoretically possible medication and dose for the disease (see step VII, comes from doctors or can be extracted directly from EMR data) as well as the cost of these drugs (see step X, come from the controlling department of a hospital).

These four results are used in the use of the system in practice (Figure 24): The "Grid" of various possible therapies as well as the new EMR data are given to the meta-model, which then generates a forecast for the possibility of a clinical event (death, thrust, readmission etc.) for different periods (1, 3, 6, 9, 12 months). These possibilities are provided together with the estimated visit free time. The cost function also recognizes the ratings of the criteria and the costs of the respective therapies as inputs and calculates for each potential therapy from the grid a "treatment rating score" on the basis of which the therapies are evaluated and the best three therapies are suggested to the doctor.

Although the present invention has been disclosed in the form of preferred embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

**Claims**

1. Method for creating predictive models (PM) for an automated clinical decision support system (CDSS) for automated supervised and semi-supervised classification and treatment optimization of clinical events using EMR data (EL,EU), comprising the steps:

- providing a number of EMR-datasets (EL,EU) comprising measurements and patient related data of a number of follow-ups,
- if necessary: treating the EMR-datasets (EL,EU) in order to estimate missing values and/or to correct outliers and/or to model temporality of measurements
- optionally: forming subgroups of related follow ups within the EMR-datasets (EL,EU),
- providing a target-variable or extract a target-variable from the EMR-dataset (EL,EU),
- providing a number of untrained predictive models (PM) which can output probabilities and assign weights to EMR-data of the EMR-datasets (EL,EU), wherein each predictive model (PM) is capable of being trained with data using methods of machine learning,
- providing a number of different time horizons,
- performing a nested cross-validation for each time horizon and for each predictive model (PM),
- selecting a predictive model (PM) for each time horizon based on the nested cross-validation.

**2.** Method as claimed in claim 1, wherein

- missing values in the EMR-datasets (EL,EU) are estimated by using a sliding weighting function, wherein especially missing values are estimated from measurements of the EMR-datasets (EL,EU) in a relevancy time window as a sum of weighted past known values, preferably by normalizing this sum by the sum of weights, and/or
- the temporality of measurements in the EMR-datasets (EL,EU) is modeled explicitly by value aggregation, wherein in order to fully account for the temporality in the data, known measurements are preferably replaced by the aggregated values,

and wherein then, a binarization approach is preferably applied to encode the potentially remaining missing values as binary zero vectors.

**3.** Method as claimed in one of the preceding claims,

- wherein the subgroups of related data from different follow-ups within the EMR datasets (EL,EU) are formed by using a clustering algorithm, wherein these subgroups are preferably exploited to generate predictors, particularly by clustering follow-ups of patients in the EMR-datasets (EL,EU) to subgroups,
- wherein the optimal number of subgroups for a given EMR-dataset (EL,EU) is determined,
- and wherein the subgroups are used as predictors in the classification of clinical events in that for each follow-up

- a first feature is created from the cluster membership of the follow-up,
- other features are created from the Minkowski distance of the follow-up to the center of each subgroup.

**4.** Method as claimed in one of the preceding claims, wherein the number of data dimensions in the EMR-datasets (EL,EU) is reduced and the EMR-datasets (EL,EU) in reduced number of dimensions are visualized,
preferably wherein the number of data dimensions in the EMR-datasets (EL,EU) is reduced by using a Principal Component Analysis ("PCA"),
especially wherein patient data of the EMR-datasets (EL,EU) are represented in a PCA-space using two or three dimensions and
preferably wherein data from follow-ups that are associated with a clinical event are represented in the PCA-space as points of one individual subgroup,
preferably wherein the subgroups are visualized, especially in a space, where dangerous areas are marked.

**5.** Method as claimed in one of the preceding claims, wherein the predictive models are selected from the group comprising logistic regression, linear discriminant analysis, quadratic discriminant analysis, decision tree, extra tree classifier, random forest, adaboost, gradient boost, bagging classifier, k-nearest neighbor, naive Bayes classifier and support vector machine.

**6.** Method as claimed in one of the preceding claims,

- wherein for each iteration of the outer loop of the nested cross-validation a set of hyperparameters relevant for the predictive models (PM), wherein it is preferred to create a grid of values for a number of the most influential hyperparameters of each predictive model (PM),
- wherein for each predefined set of hyperparameters a predictive model (PM) is trained in the inner loop of the nested cross-validation using labeled EMR-data (EL) of the EMR-datasets of the current outer loop's iteration,
- followed by the steps:

- select the optimal hyperparameter set for the given predictive model (PM),
- train the inner loop final model using all labeled EMR-data (EL) of this outer loop's iteration and selected optimal hyperparameters,
- test the inner loop optimal predictive model (PM) using labeled test data of this outer loop's iteration,

- aggregate test results of the outer loop iterations, preferably by computing the mean and standard deviation of the selected performance metrics over all outer loop's iterations,
- compare aggregated testing results of the outer loop for different trained predictive models (PM) and select the optimal predictive model (PM),
- train the final predictive model (PM) for a time horizon by repeating iterations of the outer loop for each predictive model (PM) using all labeled EMR-data (EL),
wherein preferably
- for each outer fold of the outer nested cross-validation loop a grid search is performed in the inner loop using labeled training data only to find the best supervised model,
- the best model is retrained and then evaluated on the labeled test set of the outer fold, wherein this procedure is performed a number of times, once for each of the number of iterations of the outer nested cross-validation loop,

**7.** Method as claimed in one of the preceding claims, wherein in the case that the EMR-datasets (EL,EU) comprise unlabeled EMR-data (EU), the inner loop optimal model of the nested cross-validation is retrained using a Contrastive Pessimistic Likelihood Estimation framework assigning soft-labels to unlabeled EMR-data (EU),
wherein for labeling unlabeled EMR-data (EU) of the EMR-datasets (EL,EU) the following steps are preferably applied:

- create a Supervised classifier by training and/or tuning based on labeled EMR-data (EL) from the EMR-dataset (EL,EU) and a predefined grid of hyperparameter values, by using the inner loop of a nested cross-validation routine,
- choosing soft-labels for unlabeled EMR-data (EU) randomly from a given interval of values,
- create a semi-supervised model $\theta_{semi}$ by maximizing a CPL-function of the Contrastive Pessimistic Likelihood Estimation framework (CPLE) which includes a supervised model trained on the labeled data (EL),
- using the semi-supervised model $\theta_{semi}$ for updating the randomly chosen soft-labels, in that the CPL-value is maximized,
- repeating the steps concerning the CPL-function and the CPL-value until convergence occurs.

**8.** Prediction-unit (P) for creating prediction-data for an automated clinical decision support system (CDSS) for automated supervised and semi-supervised classification and treatment optimization of clinical events using EMR datasets (EL,EU) comprising a number of trained prediction models (PM) for different time horizons as claimed in one of the preceding claims.

**9.** Method for automated clinical decision support for automated supervised and semi-supervised classification and treatment optimization of clinical events using EMR datasets (EL,EU), comprising the steps:

- providing a number of trained prediction models (PM) for a number of time horizons preferably trained by a method as claimed in one of claims 1 to 7,
- providing an EMR-dataset (EL,EU) of a patient comprising measurements and patient related data of a number of follow-ups including the data of a present patient follow-up,
- optional: treating the EMR-dataset (EL,EU) in order to estimate missing values and/or to correct outliers and/or to model temporality of measurements,
- optional: form subgroups of related data given at different follow ups within the EMR-dataset (EL,EU) and extract cluster features,
- optional: reducing the number of data dimensions in the EMR-dataset (EL,EU) and visualizing data of the EMR-dataset (EL,EU) in reduced number of dimensions,
- calculating the probability of a clinical event in all relevant time horizons, with the number of trained prediction models (PM).

**10.** Method as claimed in claim 9, comprising the additional steps:

- calculating and visualizing the probability of a clinical event for different medication and/or doses with the number of trained prediction models (PM) for each time horizon,
and/or
- estimating the time when no follow-up is necessary based on the calculation(s) of the probability of a clinical event occurring,
and preferably

- obtaining the financial costs for a therapy, especially the financial costs for medication and/or doses and/or a follow-up.

11. Method as claimed in claim 9 or 10, comprising additional steps:

- computing ratings of included criteria using a ranking algorithm from pairwise comparisons of those criteria as provided by multiple clinicians,
- optionally: normalize financial costs of various possible treatments, preferably in a way that the most expensive treatment correspond to the normalized expense of 1 and the zero dose corresponds to the normalized expense 0,
- optionally: computing 1-normalized visit-free time, wherein preferably normalized visit-free time is normalized to the interval [0,1] in the following way:

a) if the event probability is high already at the shortest relevant time horizon, then the longest "safe" normalized visit-free time is 0 for the given treatment,
b) if however the predictions for all relevant time horizons are small (e.g. < 50%), the longest visit-free period is 1 for the given treatment,

- optionally: computing a Treatment Rating Score for each plausible treatment as a sum of products of computed ratings for relevant criteria and quantitative values of those criteria.

12. Clinical Decision Support System (CDSS) for automated supervised and semi-supervised classification and treatment optimization of clinical events using EMR datasets (EL,EU), comprising a prediction-unit (P) as claimed in claim 8, wherein the Clinical Decision Support System (CDSS) is designed to execute a method as claimed in one of the claims 9 to 11 and to visualize the calculated results.

13. Using one or more of the following methods for treating EMR-datasets (EL,EU) and/or for obtaining results from EMR-datasets (EL,EU):

a) estimate missing values in the EMR-datasets (EL,EU) by using a sliding weighting function, wherein especially missing values are estimated from measurements of the EMR-datasets (EL,EU) in a relevancy time window as a sum of weighted past known values, preferably by normalizing this sum by the sum of weights,
and/or
b) model the temporality of measurements in the EMR-datasets (EL,EU) explicitly by value aggregation, wherein in order to fully account for the temporality in the data, known measurements are preferably replaced by the aggregated values,
and/or
c) form subgroups of related data from different follow-ups within the EMR datasets (EL,EU) by using a clustering algorithm,

- wherein the optimal number of subgroups for a given EMR-dataset (EL,EU) is determined,
- and wherein the subgroups are used as predictors in the classification of clinical events in that for each follow-up
- a first feature is created from the cluster membership of the follow-up,
- other features are created from the Minkowski distance of the follow-up to the center of each subgroup,

and/or
d) reduce the number of data dimensions in the EMR-datasets (EL,EU) and the EMR-datasets (EL,EU) in reduced number of dimensions are visualized,
and/or
e) performing a nested cross-validation,

- wherein for each iteration of the outer loop of the nested cross-validation a set of hyperparameters relevant for the predictive models (PM), wherein it is preferred to create a grid of values for a number of the most influential hyperparameters of each predictive model (PM),
- wherein for each predefined set of hyperparameters a predictive model (PM) is trained in the inner loop of the nested cross validation using labeled EMR-data (EL,EU) of the current outer loop's iteration,
- followed by the steps:

- select the optimal hyperparameter set for the given predictive model (PM),
- train the inner loop final model using all labeled EMR-data (EL) of this outer loop's iteration and selected optimal hyperparameters,
- test the inner loop optimal predictive model (PM) using labeled test data of this outer loop's iteration,
- aggregate test results of the outer loop iterations, preferably by computing the mean and standard deviation of the selected performance metrics over all outer loop's iterations,
- compare aggregated testing results of the outer loop for different trained predictive models (PM) and select the optimal predictive model (PM),
- train the final predictive model (PM) for a time horizon by repeating iterations of the outer loop for each predictive model (PM) using all labeled EMR-data (EL) and all unlabeled EMR-data (EU),

and/or

f) in the case that the EMR-datasets (EL,EU) comprise unlabeled EMR-data (EU), retrain the inner loop optimal model of the nested cross validation using a Contrastive Pessimistic Likelihood Estimation framework assigning soft labels to unlabeled EMR-data (EU), wherein for labeling unlabeled EMR-data (EU) of the EMR-datasets (EL,EU) the following steps are preferably applied:

- create a Supervised classifier by training and/or tuning based on labeled EMR-data (EL) from the EMR-dataset (EL,EU) and a predefined grid of hyperparameter values, by using the inner loop of a nested cross validation routine,
- choosing soft-labels for unlabeled EMR-data (EU) randomly from a given interval of values,
- create a semi-supervised model $\theta_{semi}$ by maximizing a CPL-function of the Contrastive Pessimistic Likelihood Estimation framework (CPLE) which includes a supervised model trained on the labeled data (EL),
- using the semi-supervised model $\theta_{semi}$ for updating the randomly chosen soft-labels, in that the CPL-value is maximized,
- repeating the steps concerning the CPL-function and the CPL-value until convergence occurs,

and/or

g) calculate and especially visualize the probability of a clinical event in all relevant time horizons, with the number of trained prediction models (PM), especially for different medication and/or doses with the number of trained prediction models (PM) for each time horizon,
and/or

h) estimate the time when no follow-up is necessary based on the calculation(s) of the probability of a clinical event occurring,
and/or

i) obtain the financial costs for a therapy, especially the financial costs for medication and/or doses and/or a follow-up,
and/or

j) compute ratings of included criteria using a ranking algorithm from pairwise comparisons of those criteria as provided by multiple clinicians, and

- preferably normalize financial costs of various possible treatments, preferably in a way that the most expensive treatment correspond to the normalized expense of 1 and the zero dose corresponds to the normalized expense 0,
and/or

- preferably compute 1-normalized visit-free time, wherein preferably normalized visit-free time is normalized to the interval [0,1] in the following way:

a) if the event probability is high already at the shortest relevant time horizon, then the longest "safe" normalized visit-free time is 0 for the given treatment,
b) if however the predictions for all relevant time horizons are small (e.g. < 50%), the longest visit-free period is 1 for the given treatment,

and/or

- preferably compute a Treatment Rating Score for each plausible treatment as a sum of products of computed ratings for relevant criteria and quantitative values of those criteria.

**14.** A computer program product comprising a computer program that is directly loadable into a memory of a control unit of a computer system and which comprises program elements for performing steps of the method according to any of claims 1 to 7 and/or 9 to 11 when the computer program is executed by the control unit of the computer system.

15. A computer-readable medium on which is stored program elements that can be read and executed by a computer unit in order to perform steps of the method according to any of claims 1 to 7 and/or 9 to 11 when the program elements are executed by the computer unit.

**Amended claims in accordance with Rule 137(2) EPC.**

1. Method for creating predictive models (PM) for an automated clinical decision support system (CDSS) for automated supervised and semi-supervised classification and treatment optimization of clinical events using EMR data (EL,EU), comprising the steps:

    - providing a number of EMR-datasets (EL,EU) comprising measurements and patient related data of a number of follow-ups,
    - if necessary: treating the EMR-datasets (EL,EU) in order to estimate missing values and/or to correct outliers and/or to model temporality of measurements
    - optionally: forming subgroups of related follow ups within the EMR-datasets (EL,EU),
    - providing a target-variable or extract a target-variable from the EMR-dataset (EL,EU),
    - providing a number of untrained predictive models (PM) which can output probabilities and assign weights to EMR-data of the EMR-datasets (EL,EU), wherein each predictive model (PM) is capable of being trained with data using methods of machine learning,
    - providing a number of different time horizons,
    - performing a nested cross-validation for each time horizon and for each predictive model (PM),
    - selecting a predictive model (PM) for each time horizon based on the nested cross-validation.

2. Method as claimed in claim 1, wherein

    - missing values in the EMR-datasets (EL,EU) are estimated by using a sliding weighting function, wherein especially missing values are estimated from measurements of the EMR-datasets (EL,EU) in a relevancy time window as a sum of weighted past known values, preferably by normalizing this sum by the sum of weights, and/or
    - the temporality of measurements in the EMR-datasets (EL,EU) is modeled explicitly by value aggregation, wherein in order to fully account for the temporality in the data, known measurements are preferably replaced by the aggregated values,

    and wherein then, a binarization approach is preferably applied to encode the potentially remaining missing values as binary zero vectors.

3. Method as claimed in one of the preceding claims,

    - wherein the subgroups of related data from different follow-ups within the EMR datasets (EL,EU) are formed by using a clustering algorithm, wherein these subgroups are preferably exploited to generate predictors, particularly by clustering follow-ups of patients in the EMR-datasets (EL,EU) to subgroups,
    - wherein the optimal number of subgroups for a given EMR-dataset (EL,EU) is determined,
    - and wherein the subgroups are used as predictors in the classification of clinical events in that for each follow-up

        - a first feature is created from the cluster membership of the follow-up,
        - other features are created from the Minkowski distance of the follow-up to the center of each subgroup.

4. Method as claimed in one of the preceding claims, wherein the number of data dimensions in the EMR-datasets (EL,EU) is reduced and the EMR-datasets (EL,EU) in reduced number of dimensions are visualized, preferably wherein the number of data dimensions in the EMR-datasets (EL,EU) is reduced by using a Principal Component Analysis ("PCA"), especially wherein patient data of the EMR-datasets (EL,EU) are represented in a PCA-space using two or three dimensions and preferably wherein data from follow-ups that are associated with a clinical event are represented in the PCA-space as points of one individual subgroup,
preferably wherein the subgroups are visualized, especially in a space, where dangerous areas are marked.

5. Method as claimed in one of the preceding claims, wherein the predictive models are selected from the group comprising logistic regression, linear discriminant analysis, quadratic discriminant analysis, decision tree, extra tree classifier, random forest, adaboost, gradient boost, bagging classifier, k-nearest neighbor, naive Bayes classifier and support vector machine.

6. Method as claimed in one of the preceding claims,

   - wherein for each iteration of the outer loop of the nested cross-validation a set of hyperparameters relevant for the predictive models (PM), wherein it is preferred to create a grid of values for a number of the most influential hyperparameters of each predictive model (PM),
   - wherein for each predefined set of hyperparameters a predictive model (PM) is trained in the inner loop of the nested cross-validation using labeled EMR-data (EL) of the EMR-datasets of the current outer loop's iteration,
   - followed by the steps:

      - select the optimal hyperparameter set for the given predictive model (PM),
      - train the inner loop final model using all labeled EMR-data (EL) of this outer loop's iteration and selected optimal hyperparameters,
      - test the inner loop optimal predictive model (PM) using labeled test data of this outer loop's iteration,
      - aggregate test results of the outer loop iterations, preferably by computing the mean and standard deviation of the selected performance metrics over all outer loop's iterations,
      - compare aggregated testing results of the outer loop for different trained predictive models (PM) and select the optimal predictive model (PM),
      - train the final predictive model (PM) for a time horizon by repeating iterations of the outer loop for each predictive model (PM) using all labeled EMR-data (EL), wherein preferably
      - for each outer fold of the outer nested cross-validation loop a grid search is performed in the inner loop using labeled training data only to find the best supervised model,
      - the best model is retrained and then evaluated on the labeled test set of the outer fold, wherein this procedure is performed a number of times, once for each of the number of iterations of the outer nested cross-validation loop,

7. Method as claimed in one of the preceding claims, wherein in the case that the EMR-datasets (EL,EU) comprise unlabeled EMR-data (EU), the inner loop optimal model of the nested cross-validation is retrained using a Contrastive Pessimistic Likelihood Estimation framework assigning soft-labels to unlabeled EMR-data (EU),
wherein for labeling unlabeled EMR-data (EU) of the EMR-datasets (EL,EU) the following steps are preferably applied:

   - create a Supervised classifier by training and/or tuning based on labeled EMR-data (EL) from the EMR-dataset (EL,EU) and a predefined grid of hyperparameter values, by using the inner loop of a nested cross-validation routine,
   - choosing soft-labels for unlabeled EMR-data (EU) randomly from a given interval of values,
   - create a semi-supervised model $\theta_{semi}$ by maximizing a CPL-function of the Contrastive Pessimistic Likelihood Estimation framework (CPLE) which includes a supervised model trained on the labeled data (EL),
   - using the semi-supervised model $\theta_{semi}$ for updating the randomly chosen soft-labels, in that the CPL-value is maximized,
   - repeating the steps concerning the CPL-function and the CPL-value until convergence occurs.

8. Prediction-unit (P) for creating prediction-data for an automated clinical decision support system (CDSS) for automated supervised and semi-supervised classification and treatment optimization of clinical events using EMR datasets (EL,EU) comprising a number of trained prediction models (PM) for different time horizons as claimed in one of the preceding claims.

9. Method for automated clinical decision support for automated supervised and semi-supervised classification and treatment optimization of clinical events using EMR datasets (EL,EU), comprising the steps:

   - providing a number of trained prediction models (PM) for a number of time horizons preferably trained by a method as claimed in one of claims 1 to 7,

- providing an EMR-dataset (EL,EU) of a patient comprising measurements and patient related data of a number of follow-ups including the data of a present patient follow-up,
- optional: treating the EMR-dataset (EL,EU) in order to estimate missing values and/or to correct outliers and/or to model temporality of measurements,
- optional: form subgroups of related data given at different follow ups within the EMR-dataset (EL,EU) and extract cluster features,
- optional: reducing the number of data dimensions in the EMR-dataset (EL,EU) and visualizing data of the EMR-dataset (EL,EU) in reduced number of dimensions,
- calculating the probability of a clinical event in all relevant time horizons, with the number of trained prediction models (PM).

10. Method as claimed in claim 9, comprising the additional steps:

- calculating and visualizing the probability of a clinical event for different medication and/or doses with the number of trained prediction models (PM) for each time horizon,
and/or
- estimating the time when no follow-up is necessary based on the calculation(s) of the probability of a clinical event occurring,
and preferably
- obtaining the financial costs for a therapy, especially the financial costs for medication and/or doses and/or a follow-up.

11. Method as claimed in claim 9 or 10, comprising additional steps:

- computing ratings of included criteria using a ranking algorithm from pairwise comparisons of those criteria as provided by multiple clinicians,
- optionally: normalize financial costs of various possible treatments, preferably in a way that the most expensive treatment correspond to the normalized expense of 1 and the zero dose corresponds to the normalized expense 0,
- optionally: computing 1-normalized visit-free time,
wherein preferably normalized visit-free time is normalized to the interval [0,1] in the following way:

a) if the event probability is high already at the shortest relevant time horizon, then the longest "safe" normalized visit-free time is 0 for the given treatment,
b) if however the predictions for all relevant time horizons are small (e.g. < 50%), the longest visit-free period is 1 for the given treatment,

- optionally: computing a Treatment Rating Score for each plausible treatment as a sum of products of computed ratings for relevant criteria and quantitative values of those criteria.

12. Clinical Decision Support System (CDSS) for automated supervised and semi-supervised classification and treatment optimization of clinical events using EMR datasets (EL,EU), comprising a prediction-unit (P) as claimed in claim 8, wherein the Clinical Decision Support System (CDSS) is designed to execute a method as claimed in one of the claims 9 to 11 and to visualize the calculated results.

13. Using one or more of the following methods for treating EMR-datasets (EL,EU) and/or for obtaining results from EMR-datasets (EL,EU) in the course of an automated clinical decision support system (CDSS) for automated supervised and semi-supervised classification and treatment optimization of clinical events:

a) estimate missing values in the EMR-datasets (EL,EU) by using a sliding weighting function, wherein especially missing values are estimated from measurements of the EMR-datasets (EL,EU) in a relevancy time window as a sum of weighted past known values, preferably by normalizing this sum by the sum of weights,
and/or
b) model the temporality of measurements in the EMR-datasets (EL,EU) explicitly by value aggregation, wherein in order to fully account for the temporality in the data, known measurements are preferably replaced by the aggregated values,
and/or

c) form subgroups of related data from different follow-ups within the EMR datasets (EL,EU) by using a clustering algorithm,

- wherein the optimal number of subgroups for a given EMR-dataset (EL,EU) is determined,
- and wherein the subgroups are used as predictors in the classification of clinical events in that for each follow-up
- a first feature is created from the cluster membership of the follow-up,
- other features are created from the Minkowski distance of the follow-up to the center of each subgroup,

and/or

d) reduce the number of data dimensions in the EMR-datasets (EL,EU) and the EMR-datasets (EL,EU) in reduced number of dimensions are visualized,

and/or

e) performing a nested cross-validation,

- wherein for each iteration of the outer loop of the nested cross-validation a set of hyperparameters relevant for the predictive models (PM), wherein it is preferred to create a grid of values for a number of the most influential hyperparameters of each predictive model (PM),
- wherein for each predefined set of hyperparameters a predictive model (PM) is trained in the inner loop of the nested cross validation using labeled EMR-data (EL,EU) of the current outer loop's iteration,
- followed by the steps:

- select the optimal hyperparameter set for the given predictive model (PM),
- train the inner loop final model using all labeled EMR-data (EL) of this outer loop's iteration and selected optimal hyperparameters,
- test the inner loop optimal predictive model (PM) using labeled test data of this outer loop's iteration,
- aggregate test results of the outer loop iterations, preferably by computing the mean and standard deviation of the selected performance metrics over all outer loop's iterations,
- compare aggregated testing results of the outer loop for different trained predictive models (PM) and select the optimal predictive model (PM),
- train the final predictive model (PM) for a time horizon by repeating iterations of the outer loop for each predictive model (PM) using all labeled EMR-data (EL) and all unlabeled EMR-data (EU),

and/or

f) in the case that the EMR-datasets (EL,EU) comprise unlabeled EMR-data (EU), retrain the inner loop optimal model of the nested cross validation using a Contrastive Pessimistic Likelihood Estimation framework assigning soft labels to unlabeled EMR-data (EU), wherein for labeling unlabeled EMR-data (EU) of the EMR-datasets (EL,EU) the following steps are preferably applied:

- create a Supervised classifier by training and/or tuning based on labeled EMR-data (EL) from the EMR-dataset (EL,EU) and a predefined grid of hyperparameter values, by using the inner loop of a nested cross validation routine,
- choosing soft-labels for unlabeled EMR-data (EU) randomly from a given interval of values,
- create a semi-supervised model $\theta_{semi}$ by maximizing a CPL-function of the Contrastive Pessimistic Likelihood Estimation framework (CPLE) which includes a supervised model trained on the labeled data (EL),
- using the semi-supervised model $\theta_{semi}$ for updating the randomly chosen soft-labels, in that the CPL-value is maximized,
- repeating the steps concerning the CPL-function and the CPL-value until convergence occurs,

and/or

g) calculate and especially visualize the probability of a clinical event in all relevant time horizons, with the number of trained prediction models (PM), especially for different medication and/or doses with the number of trained prediction models (PM) for each time horizon,

and/or

h) estimate the time when no follow-up is necessary based on the calculation(s) of the probability of a clinical event occurring,

and/or

i) obtain the financial costs for a therapy, especially the financial costs for medication and/or doses and/or a follow-up,

and/or

j) compute ratings of included criteria using a ranking algorithm from pairwise comparisons of those criteria as provided by multiple clinicians, and

- preferably normalize financial costs of various possible treatments, preferably in a way that the most expensive treatment correspond to the normalized expense of 1 and the zero dose corresponds to the normalized expense 0,

and/or

- preferably compute 1-normalized visit-free time, wherein preferably normalized visit-free time is normalized to the interval [0,1] in the following way:

a) if the event probability is high already at the shortest relevant time horizon, then the longest "safe" normalized visit-free time is 0 for the given treatment,

b) if however the predictions for all relevant time horizons are small (e.g. < 50%), the longest visit-free period is 1 for the given treatment,

and/or

- preferably compute a Treatment Rating Score for each plausible treatment as a sum of products of computed ratings for relevant criteria and quantitative values of those criteria.

14. A computer program product comprising a computer program that is directly loadable into a memory of a control unit of a computer system and which comprises program elements for performing steps of the method according to any of claims 1 to 7 and/or 9 to 11 when the computer program is executed by the control unit of the computer system.

15. A computer-readable medium on which is stored program elements that can be read and executed by a computer unit in order to perform steps of the method according to any of claims 1 to 7 and/or 9 to 11 when the program elements are executed by the computer unit.

# FIG 1  PRIOR ART

|  |  | prescribed medications | | | laboratory analysis | | | | | | |
|---|---|---|---|---|---|---|---|---|---|---|---|
| Patient ID | Date | Cyclosporin | Furosemide | ... | Creatinine High | Creatinine Normal | Creatinine Low | Leukocytes High | Leukocytes Normal | Leukocytes Low | ... |
| 23 | 10.05.2006 | 1 | 0 | | 1 | 0 | 0 | 0 | 1 | 0 | |
| 23 | 15.10.2006 | 1 | 0 | | 0 | 1 | 0 | 0 | 1 | 0 | |
| 57 | 10.04.2003 | 1 | 1 | | 0 | 1 | 0 | 0 | 0 | 0 | |

# FIG 2  PRIOR ART

## FIG 3    PRIOR ART

Green = Normal, Orange = Warning, Red = Alarm

## FIG 4    PRIOR ART

```
┌─────────────────────────────────────┐
│            Training set              │
└─────────────────────────────────────┘
```

Training folds          Test fold

1<sup>st</sup> iteration  ⟹ $E_1$

2<sup>nd</sup> iteration  ⟹ $E_2$

3<sup>rd</sup> iteration  ⟹ $E_3$

⋯

10<sup>th</sup> iteration  ⟹ $E_{10}$

$$E = \frac{1}{10} \sum_{i=1}^{10} E_t$$

## FIG 5    PRIOR ART

Training folds          Test fold

Outer loop

Train with optimal parameters

Training fold    Validation fold

Inner loop
Tune parameters

# FIG 6

LabValue

W

V

M

Now

Time

Weight

1

0

$\Longrightarrow$

Time

Relevancy time window of
length $n$

$$\text{LabValue(Now)} = \frac{\sum_{t=1}^{n} w_t \cdot \text{LabValue}_t}{\sum_{t=1}^{n} w_t}$$

# FIG 7

Aggregated LabValue

Median

Time

Time point of a missing measurement outside of the relevancy time window = this measurement remains missing

| Agg. Lab Value (e.g CRP in mg/L) |
| --- |
| 1.7 |
| 4 |
| 2 |
| 3.7 |
| 4.7 |
| Missing |

| Binarized Agg. Lab Value LOW | Binarized Agg. Lab Value HIGH |
| --- | --- |
| 1 | 0 |
| 0 | 1 |
| 1 | 0 |
| 0 | 1 |
| 0 | 1 |
| 0 | 0 |

# FIG 8

| EMR Var1 | EMR Var2 | ... | Cluster membership variable (RED or BLUE) | Distance from the RED cluster conter | Distance from the BLUE cluster conter |
|---|---|---|---|---|---|
| 2 | 4 | ... | 1 | 0.3003 | 9.8240 |
| 3 | 2 | ... | 1 | 0.4171 | 5.1465 |
| 2 | 1 | ... | 0 | 2.8905 | 1.4624 |
| 1 | 5 | ... | 0 | 4.3762 | 2.9551 |
| 3 | 6 | ... | 0 | 6.0328 | 2.9061 |
| 1 | 2 | ... | 1 | 0.6905 | 3.8633 |
| 1 | 4 | ... | 0 | 5.3063 | 3.7929 |
| 7 | 5 | ... | 0 | 2.7063 | 0.9288 |
| ... | ... | ... | ... | ... | ... |

EP 3 573 068 A1

## FIG 9

Scatter of patient visits in the 2D PCA space

## FIG 10

# FIG 11

ITERATION 1

Labeled EMR Data — EL

$OF_{tr}$          $OF_{tr}$          OTF

| Outer training folds | Outer test fold |

$IF_{tr}$ — | Inner training fold | Inner validation fold | — $IF_v$

$IF_{vn}$ — | Inner validation fold | Inner training fold | — $IF_{trn}$

Supervised classifier training and tuning → Trained predictive model

Model performance in iteration 1: e.g. AUC and/or F1-score

# FIG 12

EL

$OF_{tr}$     $OF_{tr}$     $OF_{tr}$     $OF_{tr}$     OTF

FIG 13

$IF_{tr}$
$IF_v$

$IF_{vn}$
$IF_{trn}$

FIG 14

$OF_{tr}$   $OF_{tr}$   $OF_{tr}$   OTF   $OF_{tr}$

FIG 15

$OF_{tr}$   $OF_{tr}$   OTF   $OF_{tr}$   $OF_{tr}$

# FIG 16

ITERATION 1

Labeled EMR Data — EL

OF$_{tr}$ · · · OF$_{tr}$ · · · OTF

| | Outer training folds | | Outer test fold |

IF$_{tr}$ — | Inner training fold | Inner validation fold | — IF$_v$

IF$_{vn}$ — | Inner validation fold | Inner training fold | — IF$_{trn}$

Supervised classifier training and tuning

EU

Unlabeled EMR Data → Contrastive Pessimistic Likelihood Estimation framework → Trained predictive model

Model performance in iteration 1: e.g. AUC and/or F1-score

EP 3 573 068 A1

## FIG 17

Patient 8, Survival prob. during follow-up

## FIG 18

**FIG 19**

| Medication name | ATC code | Dose | Prob(Event within X months) | Normalized expense | 1-normalized visit-free time | Treatment rating score | Recom-mendation |
|---|---|---|---|---|---|---|---|
| Aminosalicylic acid and similar agents | A07EC | 0% | | 0.0 | 1 | 6.8 | 9 |
| Aminosalicylic acid and similar agents | A07EC | 25% | | 0.2 | 1 | 6.6 | 8 |
| Aminosalicylic acid and similar agents | A07EC | 50% | | 0.4 | 0.6 | 3.5 | 4 |
| Aminosalicylic acid and similar agents | A07EC | 75% | | 0.6 | 0.6 | 2.2 | 1 |
| Aminosalicylic acid and similar agents | A07EC | 100% | | 0.8 | 0 | 3.3 | 3 |
| ... | ... | ... | ... | ... | | | |
| Selective immuno-suppres sants | L04AA | 0% | | 0.0 | 1 | 6.9 | 10 |
| Selective immuno-suppres sants | L04AA | 25% | | 0.25 | 0.67 | 3.6 | 5 |
| Selective immuno-suppres sants | L04AA | 50% | | 0.5 | 0.7 | 2.5 | 2 |
| Selective immuno-suppres sants | L04AA | 75% | | 0.75 | 0 | 3.8 | 6 |
| Selective immuno-suppres sants | L04AA | 100% | | 1.0 | 0 | 4.7 | 7 |

# FIG 20

**Clinician 1**

| Criterion 1 | Criterion 2 |
|:-----------:|:-----------:|
| x1 | x2 |
| x1 | x3 |
| ... | ... |
| x1 | x7 |
| ... | ... |
| x6 | x7 |

| Preferred |
|:---------:|
| x1 |
| x3 |
| ... |
| x1 |
| ... |
| x6 |

**Clinician N**

| Criterion 1 | Criterion 2 |
|:-----------:|:-----------:|
| x1 | x2 |
| x1 | x3 |
| ... | ... |
| x1 | x7 |
| ... | ... |
| x6 | x7 |

| Preferred |
|:---------:|
| x2 |
| x3 |
| ... |
| x7 |
| ... |
| x7 |

Ranking algorithm such as SVM-Rank

| Criterion | Rating $r_i$ (weights) |
|:---------:|:----------------------:|
| x1 | 1 |
| x2 | 0.38 |
| x3 | 0.23 |
| x4 | 0.78 |
| x5 | 0.64 |
| x6 | 0.91 |
| x7 | 0.11 |

# FIG 21

I

EMR data (partially missing)

EL, EU

IIa

Treating missing values and modeling temporality
- Normalized temporal aggregation
- Binarization

IIb

Treating outliers
- Expert-based
- Intersection of multiple methods

EL', EU'

III

Target and feature extraction
- Binary target variable(s) for different time horizons
- Cluster features extracted which model hidden structures

IV

Visualizing patient's "path" over time using dimensionality reduction

Aggregation used to reduce missing values and normalization to preserve typical variable ranges

State-of-the-art outlier treatment (typical in data analyses)

In addition to static and dynamic patient data from the literature, cluster features extracted

P

V

Predictive modeling
- Prob(Flares) = g (Features) Methods
- Feature Selection
- Hyperparameter optimization
- Nested X-validation AND/OR Nested X-validation with embedded CPLE framework (semi-supervised)
- Performance metrics: AUC, sensitivity, specificity, F1-score etc.

XI

Cost function optimization: computing treatment rating score
- CostFcn=f (Prob(Event), VisitFreeTime, TreatmentExpense)

Predictive models for various time horizons

PM

Prob (Event), Estimated visit free time

VI

X

Additional inputs
- Treatment expense
- Ratings (weights) of different criteria

Grid of possible treatment options, e.g. dose
- For each individual patient
- For each individual visit

Agnostic approach: automated optimat model and parameter selection

Logistic Regression
Linear Discriminant Analysis
Quadratic Discriminant Analysis
Decision Tree
Random Forest
AdaBoost
GradientBoost
BaggingClassifier
K-nearest neighbour

Actionable optimized weighted recommendations

XII

IX

Ratings

Pairwise comparisons of criteria

VII

VIII

Ranking machine learning algorithm such as SVM-Rank

**FIG 22**

I
NEW EMR data (partially missing)

IIa
Treating missing values and modeling temporality
• Normalized temporal aggregation
• Binarization

IIb
Treating outliers
• Expert-based
• Intersection of multiple methods

III
Feature extraction
• Cluster features extracted which model hidden structures

IV
Visualizing patient's "path" over time using dimensionality reduction

P
PM
VI
Trained predictive models for various time horizons

Prob (Event), Estimated visit-free time

XI
Cost function optimization: computing treatment rating score
• CostFcn = f (Prob(Event), VisitFreeTime, TreatmentExpense)

XII
Actionable optimized weighted recommendations

VII
Grid of possible teratment options, e.g. dose
• For each individual patient
• For each individual visit

EP 3 573 068 A1

# FIG 23

**I** EMR data (partially missing)

EL, EU

**IIa** Treating missing values and modeling temporality
- Normalized temporal aggregation
- Binarization

**IIb** Terating outlilers
- Expert-based
- Intersection of multiple methods

EL', EU'

**III** Target and feature extraction
- Binary target variable(s) for different time horizons
- Cluster features extracted which model hidden structures

**IV** Visualizing patient's "path" over time using dimensionality reduction

Aggregation used to reduce missing values and normalization to preserve typical variable ranges

State-of-the-art outlier treatment (typical in data analyses)

In addition to static and dynamic patient data from the literature, cluster features extracted

**P**

**V** Predictive modeling
- Prob(Flares) = g (Features) Methods
- Feature Selection
- Hyperparameter optimization
- Nested X-validation OR Nested X-validation with embedded CPLE framework (semi-supervised)
- Performance metrics: AUC, sensitivity, specificity, F1-score etc.

**PM**

**XI** Cost function from
- CostFcn = f (Prob(Event), VFisitFreeTime, TreatmentExpense)

Predictive models for various time horizons

**VI**

**X** Treatment expense

**IX** Ratings (weights) of different criteria

Grid of plausible medications and their dose

Agnostic approach automated optimal model and parameter selection

Hospital controlling/ finance

**VIII** Ranking machine learning algorithm such as SVM-Rank

**VII**

Pairwise comparisons of criteria

Clinicain

(This info can come from clinicians or it can be derived from the EMR data directly)

Logistic Regression
Linear Discrinimant Analysis
Quadratic Discriminant Analysis
Decision Tree
Random Forest
AdaBoost
GradientBoost
BaggingClassifier
K-nearest neighbour

EP 3 573 068 A1

## FIG 24

I — NEW EMR data (partially missing)

EL, EU

IIa — Treating missing values and modeling temporality
- Normalized temporal aggregation
- Binarization

IIb — Treating outliers
- Expert-based
- Intersection of multiple methods

EL', EU'

III — Feature extraction
- Cluster features extracted which model hidden structures

IV — Visualizing patient's "path" over time using dimensionality reduction

P — PM — Trained predictive models for various time horizons

Prob (Event), Estimated visit-free time

XI — Cost function optimization: computing treatment rating score
- CostFcn = f (Prob(Event), VisitFreeTime, TreatmentExpense)

VI

XII — Actionable optimized weighted recommendations for clinicians

CDSS

X — Treatment expense

VIII — Ratings (weights) of different criteria

VII — Grid of plausible medications and their dose

EP 3 573 068 A1

Europäisches
Patentamt

European
Patent Office

Office européen
des brevets

## EUROPEAN SEARCH REPORT

Application Number

EP 18 18 7263

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/073646 A1 (PEACH INTELLIHEALTH PTE LTD [SG]) 26 April 2018 (2018-04-26) | 1-3,5-15 | INV. G16H10/60 |
| Y | * paragraphs [0030], [0038], [0153], [0173], [0295]; figure 1 * | 4 | |
| X | EP 2 713 293 A2 (SIEMENS MEDICAL SOLUTIONS [US]) 2 April 2014 (2014-04-02) | 1-3,5-15 | |
| Y | * paragraphs [0007], [0038], [0059], [0073], [0076], [0080]; claims 1, 4 * | 4 | |
| X | WO 2015/157570 A1 (PARKLAND CT FOR CLINICAL INNOVATION [US]) 15 October 2015 (2015-10-15) | 1-3,5-15 | |
| Y | * paragraphs [0010], [0021] - [0025], [0064], [0055], [0131]; figure 1 * | 4 | |
| Y | US 2011/289036 A1 (STOJADINOVIC ALEXANDER [US] ET AL) 24 November 2011 (2011-11-24) * paragraphs [0002], [0041], [0082] - [0086], [0141] - [0145] * | 1-15 | |
| Y | US 2014/222719 A1 (POULIN CHRISTIAN D [US] ET AL) 7 August 2014 (2014-08-07) * paragraphs [0013], [0043], [0078] - [0081], [0089], [0102]; figure 2a * | 1-15 | TECHNICAL FIELDS SEARCHED (IPC)  G16H |
| Y | WO 2016/167923 A1 (MICROSOFT TECHNOLOGY LICENSING LLC [US]) 20 October 2016 (2016-10-20) * paragraph [0044] * | 4 | |
| Y | US 9 928 342 B1 (LABORDE DAVID [US]) 27 March 2018 (2018-03-27) * column 29, lines 35-45 * | 4 | |

The present search report has been drawn up for all claims

| Place of search | Date of completion of the search | Examiner |
|---|---|---|
| Munich | 18 February 2019 | Laub, Christoph |

EPO FORM 1503 03.82 (P04C01)

## ANNEX TO THE EUROPEAN SEARCH REPORT
## ON EUROPEAN PATENT APPLICATION NO.

EP 18 18 7263

This annex lists the patent family members relating to the patent documents cited in the above-mentioned European search report.
The members are as contained in the European Patent Office EDP file on
The European Patent Office is in no way liable for these particulars which are merely given for the purpose of information.

18-02-2019

| Patent document cited in search report | | Publication date | Patent family member(s) | | Publication date |
|---|---|---|---|---|---|
| WO 2018073646 | A1 | 26-04-2018 | NONE | | |
| EP 2713293 | A2 | 02-04-2014 | EP | 2713293 A2 | 02-04-2014 |
| | | | US | 2014088989 A1 | 27-03-2014 |
| WO 2015157570 | A1 | 15-10-2015 | CA | 2945131 A1 | 15-10-2015 |
| | | | CA | 2945134 A1 | 15-10-2015 |
| | | | CA | 2945136 A1 | 15-10-2015 |
| | | | CA | 2945137 A1 | 15-10-2015 |
| | | | CA | 2945138 A1 | 15-10-2015 |
| | | | CA | 2945143 A1 | 15-10-2015 |
| | | | EP | 3129945 A1 | 15-02-2017 |
| | | | EP | 3129949 A2 | 15-02-2017 |
| | | | EP | 3129950 A2 | 15-02-2017 |
| | | | EP | 3129951 A1 | 15-02-2017 |
| | | | WO | 2015157570 A1 | 15-10-2015 |
| | | | WO | 2015157572 A1 | 15-10-2015 |
| | | | WO | 2015157573 A2 | 15-10-2015 |
| | | | WO | 2015157575 A2 | 15-10-2015 |
| | | | WO | 2015157576 A1 | 15-10-2015 |
| | | | WO | 2015157577 A2 | 15-10-2015 |
| US 2011289036 | A1 | 24-11-2011 | US | 2011289035 A1 | 24-11-2011 |
| | | | US | 2011289036 A1 | 24-11-2011 |
| | | | US | 2011295782 A1 | 01-12-2011 |
| | | | WO | 2010045463 A2 | 22-04-2010 |
| US 2014222719 | A1 | 07-08-2014 | NONE | | |
| WO 2016167923 | A1 | 20-10-2016 | US | 2016302671 A1 | 20-10-2016 |
| | | | WO | 2016167923 A1 | 20-10-2016 |
| US 9928342 | B1 | 27-03-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **M. LOOG.** Contrasive Pessimistic Likelihood Estimation for Semi-Supervised Classification. *IEEE Transactions on pattern Analysis and Machine Intelligence,* 2016, vol. 38 (3), 462-475

- **T. JOACHIMS.** Training Linear SVMs in Linear Time. *KDD,* 20 August 2006